(19) Europäisches Patentamt
European Patent Office
Office européen des brevets

(11) EP 3 185 989 B1

(12) **EUROPÄISCHE PATENTSCHRIFT**

(45) Veröffentlichungstag und Bekanntmachung des Hinweises auf die Patenterteilung:
**11.08.2021 Patentblatt 2021/32**

(21) Anmeldenummer: **15753046.0**

(22) Anmeldetag: **20.08.2015**

(51) Int Cl.:
***B01D 53/14*** *(2006.01)*

(86) Internationale Anmeldenummer:
**PCT/EP2015/069154**

(87) Internationale Veröffentlichungsnummer:
**WO 2016/030272 (03.03.2016 Gazette 2016/09)**

# (54) ENTFERNUNG VON SCHWEFELWASSERSTOFF UND KOHLENDIOXID AUS EINEM FLUIDSTROM

REMOVAL OF HYDROGEN SULPHIDE AND CARBON DIOXIDE FROM A STREAM OF FLUID

ÉLIMINATION DU SULFURE D'HYDROGÈNE ET DU DIOXYDE DE CARBONE D'UN FLUX DE FLUIDE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **25.08.2014 EP 14182101**

(43) Veröffentlichungstag der Anmeldung:
**05.07.2017 Patentblatt 2017/27**

(60) Teilanmeldung:
**21173494.2**

(73) Patentinhaber: **BASF SE**
**67056 Ludwigshafen am Rhein (DE)**

(72) Erfinder:
- **INGRAM, Thomas**
  **68163 Mannheim (DE)**
- **NOTZ, Ralf**
  **67069 Ludwigshafen (DE)**
- **VORBERG, Gerald**
  **67346 Speyer (DE)**
- **SIEDER, Georg**
  **67098 Bad Dürkheim (DE)**

(56) Entgegenhaltungen:
**WO-A1-2005/075056 US-A- 4 471 138**
**US-A1- 2013 243 676**

- **LU J G ET AL: "Selective absorption of H2S from gas mixtures into aqueous solutions of blended amines of methyldiethanolamine and 2-tertiarybutylamino-2-ethoxyethanol in a packed column", SEPARATION AND PURIFICATION TECHNOLOGY, ELSEVIER SCIENCE, AMSTERDAM, NL, Bd. 52, Nr. 2, 1. Dezember 2006 (2006-12-01), Seiten 209-217, XP028035520, ISSN: 1383-5866, DOI: 10.1016/J.SEPPUR.2006.04.003 [gefunden am 2006-12-01] in der Anmeldung erwähnt**
- **None**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft ein Verfahren zur Entfernung von Schwefelwasserstoff und Kohlendioxid aus einem Fluidstrom, das bei ausreichender Schwefelwasserstoff-Selektivität eine verbesserte Kohlendioxid-Entfernung erlaubt.

Die Entfernung von Sauergasen, wie z. B. $CO_2$, $H_2S$, $SO_2$, $CS_2$, HCN, COS oder Merkaptanen, aus Fluidströmen wie Erdgas, Raffineriegas oder Synthesegas, ist aus unterschiedlichen Gründen von Bedeutung. Der Gehalt an Schwefelverbindungen von Erdgas muss durch geeignete Aufbereitungsmaßnahmen unmittelbar an der Erdgasquelle reduziert werden, denn die Schwefelverbindungen bilden in dem vom Erdgas häufig mitgeführten Wasser Säuren, die korrosiv wirken. Für den Transport des Erdgases in einer Pipeline oder die Weiterverarbeitung in einer Erdgasverflüssigungsanlage (LNG = liquefied natural gas) müssen daher vorgegebene Grenzwerte der schwefelhaltigen Verunreinigungen eingehalten werden. Darüber hinaus sind zahlreiche Schwefelverbindungen bereits in niedrigen Konzentrationen übel riechend und toxisch.

Kohlendioxid muss unter anderem aus Erdgas entfernt werden, weil eine hohe Konzentration an $CO_2$ den Brennwert des Gases reduziert. Außerdem kann $CO_2$ in Verbindung mit Feuchtigkeit, die in den Fluidströmen häufig mitgeführt wird, zu Korrosion an Leitungen und Armaturen führen. Wird Erdgas für den Transport zu Flüssigerdgas (LNG = Liquid Natural Gas) verflüssigt, muss das $CO_2$ zuvor weitgehend entfernt werden. Bei der Temperatur des Flüssigerdgases (etwa -162 °C) würde das $CO_2$ resublimieren und Anlagenteile beschädigen. Andererseits kann eine zu geringe Konzentration an $CO_2$ ebenfalls unerwünscht sein, z. B. bei der Einspeisung in das Erdgasnetz, da dadurch der Brennwert des Gases zu hoch sein kann.

Zur Entfernung von Sauergasen werden Wäschen mit wässrigen Lösungen anorganischer oder organischer Basen eingesetzt. Beim Lösen von Sauergasen in dem Absorptionsmittel bilden sich mit den Basen Ionen. Das Absorptionsmittel kann durch Entspannen auf einen niedrigeren Druck und/oder Strippen regeneriert werden, wobei die ionischen Spezies zu Sauergasen zurück reagieren und/oder mittels Dampf abgestrippt werden. Nach dem Regenerationsprozess kann das Absorptionsmittel wiederverwendet werden.

Ein Verfahren, bei dem alle sauren Gase, insbesondere $CO_2$ und $H_2S$, weitestgehend entfernt werden, wird als "Totalabsorption" bezeichnet. In bestimmten Fällen kann es dagegen wünschenswert sein, bevorzugt $H_2S$ vor $CO_2$ zu absorbieren, z. B. um ein Brennwert-optimiertes $CO_2/H_2S$-Verhältnis für eine nachgeschaltete Claus-Anlage zu erhalten. In diesem Fall spricht man von einer "selektiven Wäsche". Ein ungünstiges $CO_2/H_2S$-Verhältnis kann die Leistung und Effizienz der Claus-Anlage durch Bildung von $COS/CS_2$ und Verkokung des Claus-Katalysators oder durch einen zu geringen Heizwert beeinträchtigen.

Stark sterisch gehinderte sekundäre Amine (als solche werden Amine mit einem sterischen Parameter (Taft-Konstante) $E_s$ von mehr als 1,75 bezeichnet), wie 2-(2-tert-Butylaminoethoxy)ethanol, und tertiäre Amine, wie Methyldiethanolamin (MDEA), zeigen kinetische Selektivität für $H_2S$ gegenüber $CO_2$. Diese Amine reagieren nicht direkt mit $CO_2$; vielmehr wird $CO_2$ in einer langsamen Reaktion mit dem Amin und mit Wasser zu Bicarbonat umgesetzt - im Gegensatz dazu reagiert $H_2S$ in wässrigen Aminlösungen sofort. Diese Amine eignen sich daher insbesondere für eine selektive Entfernung von $H_2S$ aus Gasgemischen, die $CO_2$ und $H_2S$ enthalten.

Die selektive Entfernung von Schwefelwasserstoff findet vielfach Anwendung bei Fluidströmen mit niedrigen Sauergas-Partialdrücken, wie z. B. in Tailgas oder bei der Sauergasanreicherung (Acid Gas Enrichment, AGE), beispielsweise zur Anreicherung von $H_2S$ vor dem Claus-Prozess.

So wurde in der US 4,471,138 gezeigt, dass stark sterisch gehinderte sekundäre Amine, wie 2-(2-tert-Butylaminoethoxy)ethanol, auch in Kombination mit weiteren Aminen wie Methyldiethanolamin, eine deutlich höhere $H_2S$-Selektivität als Methyldiethanolamin aufweisen. Dieser Effekt wurde von Lu et al. in Separation and Purification Technology, 2006, 52, 209-217 bestätigt. Die EP 0 084 943 offenbart die Verwendung von stark sterisch gehinderten sekundären und tertiären Alkanolaminen in Absorptionslösungen zur selektiven Entfernung von Schwefelwasserstoff gegenüber Kohlendioxid aus Gasströmen.

Auch bei der Erdgasaufbereitung (Natural Gas Treatment) für Pipelinegas kann eine selektive Entfernung von $H_2S$ gegenüber $CO_2$ erwünscht sein. Der Absorptionsschritt bei der Erdgasaufbereitung erfolgt üblicherweise bei hohen Drücken von etwa 20 bis 130 bar (absolut) und Sauergas-Partialdrücken von z.B. mindestens 0,2 bar für $H_2S$ und mindestens 1 bar für $CO_2$, die deutlich höher sind als die Sauergas-Partialdrücke bei der Tailgas-Behandlung.

Die US 2013/0243676 beschreibt ein Verfahren zur Absorption von $H_2S$ und $CO_2$ aus einem Gasgemisch mit einem Absorptionsmittel, welches einen stark sterisch gehinderten tertiären Etheramintriethylenglykolalkohol oder Derivate davon sowie ein flüssiges Amin umfasst.

In vielen Fällen strebt man bei der Erdgasaufbereitung eine gleichzeitige Entfernung von $H_2S$ und $CO_2$ an, wobei vorgegebene $H_2S$-Grenzwerte eingehalten werden müssen, aber eine vollständige Entfernung von $CO_2$ nicht erforderlich ist. Die für Pipelinegas typische Spezifikation erfordert eine Sauergasentfernung auf etwa 1,5 bis 3,5 Vol.-% $CO_2$ und weniger als 4 vppm $H_2S$. In diesen Fällen ist eine maximale $H_2S$-Selektivität nicht erwünscht.

Der Erfindung liegt daher die Aufgabe zu Grunde, ein Verfahren anzugeben, welches einen hohen Abtrennungsgrad an Schwefelwasserstoff bei einer gleichzeitig hohen Coabsorption von Kohlendioxid erlaubt. Die erforderliche Regenerationsenergie soll gegenüber $H_2S$-selektiven Absorptionsmitteln nicht wesentlich erhöht sein.

Die Aufgabe wird gelöst durch ein Verfahren zur Entfernung von Schwefelwasserstoff und Kohlendioxid aus einem Fluidstrom, wie in Anspruch 1 dargelegt. Im Allgemeinen wird beschrieben ein Verfahren umfassend

a) einen Absorptionsschritt, bei dem man den Fluidstrom mit einem Absorptionsmittel in Kontakt bringt, das eine wässrige Lösung

(i) eines Amins der allgemeinen Formel (I)

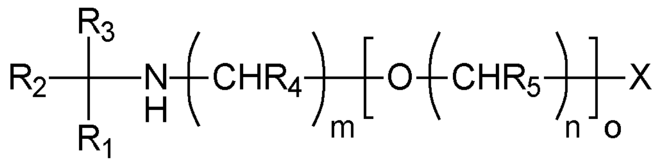

(I)

worin $R_1$, $R_2$ und $R_3$ unabhängig voneinander ausgewählt sind unter $C_{1-4}$-Alkyl und $C_{1-4}$-Hydroxyalkyl; $R_4$ jeweils unabhängig ausgewählt ist unter Wasserstoff, $C_{1-4}$-Alkyl und $C_{1-4}$-Hydroxyalkyl; $R_5$ jeweils unabhängig ausgewählt ist unter Wasserstoff, $C_{1-4}$-Alkyl und $C_{1-4}$-Hydroxyalkyl; X für OH oder $NH(CR_1R_2R_3)$ steht; m für 2, 3, 4 oder 5 steht; n für 2, 3, 4 oder 5 steht; und o für 0 oder 1 steht; und gegebenenfalls (ii) wenigstens eines tertiären Amins umfasst, wobei das molare Verhältnis von (i) zu (ii) im Bereich von 0,1 bis 0,9 liegt; wobei mindestens 90% des Schwefelwasserstoffs aus dem Fluidstrom entfernt werden und die Selektivität für Schwefelwasserstoff gegenüber Kohlendioxid nicht größer als 8 ist, wobei man ein $CO_2$- und $H_2S$-beladenes Absorptionsmittel erhält;

b) einen Regenerationsschritt, bei dem man zumindest einen Teilstrom des $CO_2$- und $H_2S$-beladenen Absorptionsmittels regeneriert und man ein regeneriertes Absorptionsmittel erhält; und

c) einen Rückführschritt, bei dem man zumindest einen Teilstrom des regenerierten Absorptionsmittels in den Absorptionsschritt a) zurückführt.

Nach dem Stand der Technik ist die $H_2S$-Selektivität von 2-(2-tert-Butylaminoethoxy)ethanol (TBAEE) bei niedrigen $H_2S$-Partialdrücken größer ist als die des tertiären Amins Methyldiethanolamin (MDEA). Es wurde nun überraschend gefunden, dass die $H_2S$-Selektivität von Aminen der Formel (I), wie TBAEE, bei hohen Sauergas-Partialdrücken abnimmt und kleiner ist als die von MDEA. Das heißt, dass Amine der Formel (I) unter diesen Bedingungen $CO_2$ schneller und damit bei gleicher Absorberhöhe mehr $CO_2$ aufnehmen können.

Im Absorptionsschritt a) ist die Selektivität für Schwefelwasserstoff gegenüber Kohlendioxid nicht größer als 8, vorzugsweise nicht größer als 6, insbesondere nicht größer als 5, besonders bevorzugt nicht größer als 4. Die Selektivität ist in der Regel größer als 1.

Unter "Selektivität für Schwefelwasserstoff" wird vorliegend der Wert des folgenden Quotienten verstanden:

$$\frac{\dfrac{y(H_2S)_{feed}-y(H_2S)_{treat}}{y(H_2S)_{feed}}}{\dfrac{y(CO_2)_{feed}-y(CO_2)_{treat}}{y(CO_2)_{feed}}}$$

worin $y(H_2S)_{feed}$ für den Stoffmengenanteil (mol/mol) von $H_2S$ im Ausgangsfluid, $y(H_2S)_{treat}$ für den Stoffmengenanteil im behandelten Fluid, $y(CO_2)_{feed}$ für den Stoffmengenanteil von $CO_2$ im Ausgangsfluid und $y(CO_2)_{treat}$ für den Stoffmengenanteil von $CO_2$ im behandelten Fluid steht.

Vorzugsweise beträgt die kumulierte $CO_2$- und $H_2S$-Beladung des $CO_2$- und $H_2S$-beladenen Absorptionsmittels nach dem Absorptionsschritt a) wenigstens 0,25 mol/mol, besonders bevorzugt wenigstens 0,30 mol/mol, ausgedrückt als Summe der Stoffmengen an im Absorptionsmittel gelöstem $CO_2$ und $H_2S$ pro Stoffmenge der Komponenten (i) + (ii).

Das Absorptionsmittel umfasst eine wässrige Lösung eines Amins der allgemeinen Formel (I),

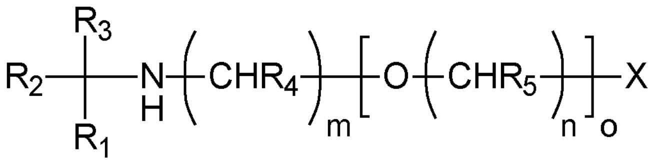

(I)

worin $R_1$, $R_2$ und $R_3$ unabhängig voneinander ausgewählt sind unter $C_{1-4}$-Alkyl und $C_{1-4}$-Hydroxyalkyl; $R_4$ jeweils unabhängig ausgewählt ist unter Wasserstoff, $C_{1-4}$-Alkyl und $C_{1-4}$-Hydroxyalkyl; $R_5$ jeweils unabhängig ausgewählt ist unter Wasserstoff, $C_{1-4}$-Alkyl und $C_{1-4}$-Hydroxyalkyl; X für OH oder $NH(CR_1R_2R_3)$ steht; m für 2, 3, 4 oder 5 steht; n für 2, 3, 4 oder 5 steht; und o für 0 oder 1 steht. $R_4$ ist in jeder Wiederholungseinheit unabhängig ausgewählt unter Wasserstoff, $C_{1-4}$-Alkyl und $C_{1-4}$-Hydroxyalkyl. $R_5$ ist in jeder Wiederholungseinheit unabhängig ausgewählt unter Wasserstoff, $C_{1-4}$-Alkyl und $C_{1-4}$-Hydroxyalkyl. Vorzugsweise stehen $R_1$, $R_2$ und $R_3$ jeweils für Methyl. $R_4$ steht vorzugsweise für Wasserstoff oder Methyl, insbesondere für Wasserstoff. $R_5$ steht vorzugsweise für Wasserstoff oder Methyl, insbesondere für Wasserstoff. Vorzugsweise steht m für 2, 3 oder 4, insbesondere 2 oder 3, ganz besonders bevorzugt für 2. Vorzugsweise steht n für 2, 3 oder 4, insbesondere 2 oder 3, ganz besonders bevorzugt für 2. Vorzugsweise steht o für 1.

Geeignete Amine der Formel (I) sind 2-(2-tert-Butylaminoethoxy)ethanol (TBAEE), 2-(2-tert-Amylaminoethoxy)ethanol, 2-(2-(1-Methyl-1-ethylpropyl-amino)ethoxy)ethanol, 2-(tert-Butylamino)ethanol, 2-(tert-Butylamino)propanol, 2-(tert-Butylamino)butanol, (2-(tert-Butylamino)ethyl)methylamin und Gemische davon. Erfindungsgemäß ist das Amin (i) 2-(2-tert-Butylamino-ethoxy)ethanol.

Die exotherme Wärmetönung der Reaktion von Aminen der Formel (I) mit Kohlendioxid ist größer als die tertiärer Amine. Bei Verwendung eines Absorptionsmittels, das ein Amin der allgemeinen Formel (I) als einzige basische Komponente enthält, kann - insbesondere bei niedrigem Absorptionsmittel/Fluidstrom-Verhältnis - die Wärmetönung zu hoch und eine gewünschte $H_2S$-Spezifikation unter Umständen nicht erreicht werden. Die Erfindung sieht daher vor, dass das Absorptionsmittel neben einem Amin (i) gegebenenfalls wenigstens ein tertiäres Amin (ii) enthalten kann. Neben der Steuerung der Wärmetönung wurde gefunden, dass durch die Zugabe eines tertiären Amins (ii) zum Amin (i) die $H_2S$-Selektivität gesteuert werden kann. Je höher der Anteil an Amin (i), desto niedriger die $H_2S$-Selektivität, d. h. bei gleicher Absorberhöhe kann mehr $CO_2$ abgetrennt werden. Gleichzeitig erlaubt das Verfahren einen hohen Abtrennungsgrad für $H_2S$.

Unter einem "tertiären Amin" werden Verbindungen mit wenigstens einer tertiären Aminogruppe verstanden. Das tertiäre Amin (ii) enthält vorzugsweise ausschließlich tertiäre Aminogruppen, d. h. es enthält neben wenigstens einer tertiären Aminogruppe keine primären oder sekundären Aminogruppen. Das tertiäre Amin (ii) verfügt vorzugsweise nicht über saure Gruppen, wie insbesondere Phosphonsäure-, Sulfonsäure- und/oder Carbonsäuregruppen.

Zu den geeigneten tertiären Aminen (ii) zählen insbesondere:

1. Tertiäre Alkanolamine wie

Bis(2-hydroxyethyl)-methylamin (Methyldiethanolamin, MDEA), Tris(2-hydroxyethyl)amin (Triethanolamin, TEA), Tributanolamin, 2-Diethylaminoethanol (Diethylethanolamin, DEEA), 2-Dimethylaminoethanol (Dimethylethanolamin, DMEA), 3-Dimethylamino-1-propanol (N,N-Dimethylpropanolamin), 3-Diethylamino-1-propanol, 2-Diisopropylaminoethanol (DIEA), N,N-Bis(2-hydroxypropyl)methylamin (Methyldiisopropanolamin, MDIPA);

2. Tertiäre Aminoether wie

3-Methoxypropyldimethylamin;

3. Tertiäre Polyamine, z. B. bistertiäre Diamine wie

N,N,N',N'-Tetramethylethylendiamin, N,N-Diethyl-N',N'-dimethylethylendiamin, N,N,N',N'-Tetraethylethylendiamin, N,N,N',N'-Tetramethyl-1,3-propandiamin (TMPDA), N,N,N',N'-Tetraethyl-1,3-propandiamin (TEPDA), N,N,N',N'-Tetramethyl-1,6-hexandiamin, N,N-Dimethyl-N',N'-diethylethylendiamin (DMDEEDA), 1-Dimethylamino-2-dimethylaminoethoxyethan (Bis[2-(dimethylamino)ethyl]ether), 1,4-Diazabicyclo[2.2.2]octan (TEDA), Tetramethyl-1,6-hexandiamin; und Gemische davon.

Tertiäre Alkanolamine, d. h. Amine mit wenigstens einer an das Stickstoffatom gebundenen Hydroxyalkylgruppe, erfindungsgemäß ist Methyldiethanolamin (MDEA).

Das molare Verhältnis von (i) zu (ii) liegt im Bereich von 0,1 bis 0,9. Durch Variation des molaren Verhältnisses von (i) zu (ii) kann die $H_2S$-Selektivität innerhalb den erfindungsgemäßen Grenzwerten den jeweiligen Anforderungen

angepasst werden. Trotz verringerter $H_2S$-Selektivität ist die Regenerationsenergie gleich der oder geringer als die eines $H_2S$-selektiven Absorptionsmittels.

Im Allgemeinen beträgt die Gesamtkonzentration von (i) und (ii) in der wässrigen Lösung 10 bis 60 Gew.-%, bevorzugt 20 bis 50 Gew.-%, besonders bevorzugt 30 bis 50 Gew.-%.

In einer Ausführungsform enthält die wässrige Lösung wenigstens ein organisches Lösungsmittel. Das organische Lösungsmittel ist bevorzugt ausgewählt unter Sulfolan, Glycolen wie Ethylenglycol, Diethylenglycol, Ethylenglycoldimethylether, Triethylenglycol, Triethylenglycoldimethylether, Di- oder Mono-($C_{1-4}$-Alkylether)-monoethylengylcolen und Di- oder Mono-($C_{1-4}$-Alkylether)-polyethylengylcolen, N-Methylpyrrolidon, N-Methyl-3-morpholin, N-Formylmorpholin, N-Acetylmorphlin, N,N-Dimethylformamid, N,N-Dimethylimidazolidin-2-on, N-Methylimidazol und Gemische davon.

Bevorzugt enthält das Absorptionsmittel kein sterisch ungehindertes primäres oder sekundäres Amin. Verbindungen dieser Art wirken als starke Promotoren der $CO_2$-Absorption. Dadurch kann die $H_2S$-Selektivität des Absorptionsmittels verloren gehen.

Unter einem sterisch ungehinderten primären oder sekundären Amin werden Verbindungen verstanden, die über primäre oder sekundäre Aminogruppen verfügen, an die lediglich Wasserstoffatome oder primäre Kohlenstoffatome gebunden sind.

Das Absorptionsmittel kann auch Additive, wie Korrosionsinhibitoren, Enzyme etc. enthalten. Im Allgemeinen liegt die Menge an derartigen Additiven im Bereich von etwa 0,01 bis 3 Gew.-% des Absorptionsmittels.

Das erfindungsgemäße Verfahren ist geeignet zur Behandlung von Fluiden aller Art. Fluide sind einerseits Gase, wie Erdgas, Synthesegas, Koksofengas, Spaltgas, Kohlevergasungsgas, Kreisgas, Deponiegase und Verbrennungsgase, und andererseits mit dem Absorptionsmittel im Wesentlichen nicht mischbare Flüssigkeiten, wie LPG (Liquefied Petroleum Gas) oder NGL (Natural Gas Liquids). Das erfindungsgemäße Verfahren ist besonders zur Behandlung von kohlenwasserstoffhaltigen Fluidströmen geeignet. Die enthaltenen Kohlenwasserstoffe sind z. B. aliphatische Kohlenwasserstoffe, wie $C_1$-$C_4$-Kohlenwasserstoffe, wie Methan, ungesättigte Kohlenwasserstoffe, wie Ethylen oder Propylen, oder aromatische Kohlenwasserstoffe wie Benzol, Toluol oder Xylol.

Das erfindungsgemäße Verfahren bzw. Absorptionsmittel ist zur Entfernung von $CO_2$ und $H_2S$ geeignet. Neben Kohlendioxid und Schwefelwasserstoff können andere saure Gase im Fluidstrom vorhanden sein, wie COS und Mercaptane. Außerdem können auch $SO_3$, $SO_2$, $CS_2$ und HCN entfernt werden.

In bevorzugten Ausführungsformen ist der Fluidstrom ein Kohlenwasserstoffe enthaltender Fluidstrom; insbesondere ein Erdgasstrom. Besonders bevorzugt enthält der Fluidstrom mehr als 1,0 Vol.-% Kohlenwasserstoffe, ganz besonders bevorzugt mehr als 5,0 Vol.-% Kohlenwasserstoffe, am meisten bevorzugt mehr als 15 Vol.-% Kohlenwasserstoffe.

Der Schwefelwasserstoff-Partialdruck im Fluidstrom beträgt mindestens 0,1 bar, insbesondere mindestens 0,5 bar, ganz besonders bevorzugt mindestens 1 bar. Der Kohlendioxid-Partialdruck im Fluidstrom beträgt mindestens 1 bar. Die angegebenen Partialdrücke beziehen sich auf den Fluidstrom beim erstmaligen Kontakt mit dem Absorptionsmittel im Absorptionsschritt.

In Ausführungsformen liegt im Fluidstrom ein Gesamtdruck von mindestens 20 bar vor. In der Regel liegt im Fluidstrom ein Gesamtdruck von höchstens 180 bar, meist höchstens 120 bar, vor. Der Gesamtdruck des Fluidstroms entspricht im Wesentlichen dem Druck im Absorber im Absorptionsschritt a).

Bei dem Absorptionsschritt a) handelt es sich nicht um eine Totalabsorption, d. h. der behandelte Fluidstrom enthält eine gegenüber der Konzentration im zu behandelnden Fluidstrom verringerte Konzentration an $CO_2$. Der behandelte Fluidstrom enthält üblicherweise mindestens noch 1,0 Vol.-% $CO_2$, bevorzugt mindestens 1,5 Vol.-% $CO_2$, besonders bevorzugt mindestens 2,0 Vol.-% $CO_2$.

Das erfindungsgemäße Verfahren bedient sich einer selektiven Entfernung von Schwefelwasserstoff gegenüber $CO_2$, wobei jedoch die Selektivität für Schwefelwasserstoff gegenüber Kohlendioxid nicht größer als 8 ist. Es werden mindestens 90% des Schwefelwasserstoffs aus dem Fluidstrom entfernt. Die prozentuale Entfernung von Schwefelwasserstoff kann durch Bilanzierung des Volumenstroms des zu behandelnden Fluidstroms (in $Nm^3$) multipliziert mit der $H_2S$-Konzentration im zu behandelnden Fluidstrom (in Vol.-%) gegen den Volumenstrom des zu behandelten Fluidstroms multipliziert mit der $H_2S$-Konzentration im zu behandelten Fluidstrom ermittelt werden.

Der Fachmann kann einen hohen Abtrennungsgrad an Schwefelwasserstoff bei einer definierten Selektivität erreichen, indem er die Bedingungen im Absorptionsschritt, wie insbesondere das Absorptionsmittel/Fluidstrom-Verhältnis, die Kolonnenhöhe des Absorbers, die Art der kontaktfördernden Einbauten im Absorber, wie Füllkörper, Böden oder Packungen, und/oder die Restbeladung des regenerierten Absorptionsmittels variiert.

Ein niedriges Absorptionsmittel/Fluidstrom-Verhältnis führt zu einer erhöhten Selektivität des Absorptionsmittels, ein höheres Absorptionsmittel/Fluidstrom-Verhältnis führt zu einer unselektiveren Absorption. Da $CO_2$ langsamer absorbiert wird als $H_2S$, wird bei einer längeren Verweilzeit mehr $CO_2$ aufgenommen als bei einer kürzeren Verweilzeit. Eine höhere Kolonne bewirkt daher eine unselektivere Absorption. Böden oder Packungen mit größerem Flüssigkeits-Holdup führen ebenfalls zu einer unselektiveren Absorption. Über die bei der Regeneration eingebrachte Erwärmungsenergie kann die Restbeladung des regenerierten Absorptionsmittels eingestellt werden. Eine geringere Restbeladung

des regenerierten Absorptionsmittels führt zu einer verbesserten Absorption.

Im erfindungsgemäßen Verfahren wird der Fluidstrom in einem Absorptionsschritt in einem Absorber in Kontakt mit dem Absorptionsmittel gebracht, wodurch Kohlendioxid und Schwefelwasserstoff zumindest teilweise ausgewaschen werden. Man erhält einen $CO_2$- und $H_2S$-abgereicherten Fluidstrom und ein $CO_2$- und $H_2S$-beladenes Absorptionsmittel.

Als Absorber fungiert eine in üblichen Gaswäscheverfahren eingesetzte Waschvorrichtung. Geeignete Waschvorrichtungen sind beispielsweise Füllkörper-, Packungs- und Bodenkolonnen, Membrankontaktoren, Radialstromwäscher, Strahlwäscher, Venturi-Wäscher und Rotations-Sprühwäscher, bevorzugt Packungs-, Füllkörper- und Bodenkolonnen, besonders bevorzugt Boden- und Füllkörperkolonnen. Die Behandlung des Fluidstroms mit dem Absorptionsmittel erfolgt dabei bevorzugt in einer Kolonne im Gegenstrom. Das Fluid wird dabei im Allgemeinen in den unteren Bereich und das Absorptionsmittel in den oberen Bereich der Kolonne eingespeist. In Bodenkolonnen sind Sieb-, Glocken- oder Ventilböden eingebaut, über welche die Flüssigkeit strömt. Füllkörperkolonnen können mit unterschiedlichen Formkörpern gefüllt werden. Wärme- und Stoffaustausch werden durch die Vergrößerung der Oberfläche aufgrund der meist etwa 25 bis 80 mm großen Formkörper verbessert. Bekannte Beispiele sind der Raschig-Ring (ein Hohlzylinder), Pall-Ring, Hiflow-Ring, Intalox-Sattel und dergleichen. Die Füllkörper können geordnet, aber auch regellos (als Schüttung) in die Kolonne eingebracht werden. Als Materialien kommen in Frage Glas, Keramik, Metall und Kunststoffe. Strukturierte Packungen sind eine Weiterentwicklung der geordneten Füllkörper. Sie weisen eine regelmäßig geformte Struktur auf. Dadurch ist es bei Packungen möglich, Druckverluste bei der Gasströmung zu reduzieren. Es gibt verschiedene Ausführungen von Packungen z. B. Gewebe- oder Blechpackungen. Als Material können Metall, Kunststoff, Glas und Keramik eingesetzt werden.

Die Temperatur des Absorptionsmittels beträgt im Absorptionsschritt im Allgemeinen etwa 30 bis 100°C, bei Verwendung einer Kolonne beispielsweise 30 bis 70°C am Kopf der Kolonne und 50 bis 100°C am Boden der Kolonne.

Das erfindungsgemäße Verfahren kann einen oder mehrere, insbesondere zwei, aufeinander folgende Absorptionsschritte umfassen. Die Absorption kann in mehreren aufeinander folgenden Teilschritten durchgeführt werden, wobei das die sauren Gasbestandteile enthaltende Rohgas in jedem der Teilschritte mit jeweils einem Teilstrom des Absorptionsmittels in Kontakt gebracht wird. Das Absorptionsmittel, mit dem das Rohgas in Kontakt gebracht wird, kann bereits teilweise mit sauren Gasen beladen sein, d. h. es kann sich beispielsweise um ein Absorptionsmittel, das aus einem nachfolgenden Absorptionsschritt in den ersten Absorptionsschritt zurückgeführt wurde, oder um teilregeneriertes Absorptionsmittel handeln. Bezüglich der Durchführung der zweistufigen Absorption wird Bezug genommen auf die Druckschriften EP 0 159 495, EP 0 190 434, EP 0 359 991 und WO 00100271.

Das Verfahren umfasst einen Regenerationsschritt, bei dem man das $CO_2$- und $H_2S$-beladene Absorptionsmittel regeneriert. Im Regenerationsschritt b) werden aus dem $CO_2$- und $H_2S$-beladenen Absorptionsmittel $CO_2$ und $H_2S$ und gegebenenfalls weitere saure Gasbestandteile freigesetzt, wobei ein regeneriertes Absorptionsmittel erhalten wird. In der Regel umfasst der Regenerationsschritt b) wenigstens eine der Maßnahmen Erwärmung, Entspannung und Strippen mit einem inerten Fluid.

Das $CO_2$- und $H_2S$-beladene Absorptionsmittel wird vorzugsweise auf eine Schwefelwasserstoffbeladung regeneriert, die einer Gleichgewichtsbeladung für einen Schwefelwasserstoffgehalt von vorzugsweise weniger als 90%, besonders bevorzugt weniger als 50%, des Schwefelwasserstoffgehalts des behandelten Fluidstroms entspricht. Unter Gleichgewichtsbeladung wird der Gehalt an Schwefelwasserstoff im Absorptionsmittel verstanden, der unter den Druck- und Temperaturbedingungen am Kopf des Absorbers im Gleichgewicht mit dem angegebenen Gehalt an Schwefelwasserstoff im behandelten Gasstrom steht, der den Absorber verlässt.

Vorzugsweise beträgt die kumulierte $CO_2$- und $H_2S$-Beladung des regenerierten Absorptionsmittels weniger als 0,20 mol/mol, insbesondere weniger als 0,15 mol/mol. Die Beladung wird ausgedrückt als Stoffmenge des im Absorptionsmittel gelösten $CO_2 + H_2S$ pro Stoffmenge der Komponenten (i) + (ii).

Der Regenerationsschritt b) umfasst bevorzugt eine Erwärmung des mit den sauren Gasbestandteilen beladenen Absorptionsmittels. Die absorbierten Sauergase werden dabei mittels des durch Erhitzens der Lösung gewonnenen Wasserdampfes abgestrippt. Anstelle des Dampfes kann auch ein inertes Fluid, wie Stickstoff, verwendet werden. Der absolute Druck im Desorber liegt normalerweise bei 0,1 bis 3,5 bar, bevorzugt 1,0 bis 2,5 bar. Die Temperatur liegt normalerweise bei 50 °C bis 170 °C, bevorzugt bei 80 °C bis 130 °C, wobei die Temperatur natürlich vom Druck abhängig ist.

Der Regenerationsschritt b) kann alternativ oder zusätzlich eine Druckentspannung umfassen. Diese beinhaltet mindestens eine Druckentspannung des beladenen Absorptionsmittels von einem höheren Druck, wie er bei der Durchführung des Absorptionsschritts herrscht, auf einen niedrigeren Druck. Die Druckentspannung kann beispielsweise mittels eines Drosselventils und/oder einer Entspannungsturbine geschehen. Die Regeneration mit einer Entspannungsstufe ist beispielsweise beschrieben in den Druckschriften US 4,537,753 und US 4,553,984.

Die Freisetzung der sauren Gasbestandteile im Regenerationsschritt b) kann beispielsweise in einer Entspannungskolonne, z. B. einem senkrecht oder waagerecht eingebauten Flash-Behälter oder einer Gegenstromkolonne mit Einbauten, erfolgen.

Bei der Regenerationskolonne kann es sich ebenfalls um eine Füllkörper-, Packungs- oder Bodenkolonne

handeln. Die Regenerationskolonne weist am Sumpf einen Aufheizer auf, z. B. einen Aufkocher, Naturumlaufverdampfer, Zwangsumlaufverdampfer, oder Zwangsumlaufentspannungsverdampfer. Am Kopf weist die Regenerationskolonne einen Auslass für die freigesetzten Sauergase auf. Mitgeführte Absorptionsmitteldämpfe können optional in einem Kondensator kondensiert und in die Kolonne zurückgeführt.

Es können mehrere Entspannungskolonnen hintereinander geschaltet werden, in denen bei unterschiedlichen Drücken regeneriert wird. Beispielsweise kann in einer Vorentspannungskolonne bei hohem Druck, der typischerweise etwa 1,5 bar oberhalb des Partialdrucks der sauren Gasbestandteile im Absorptionsschritt liegt, und in einer Hauptentspannungskolonne bei niedrigem Druck, beispielsweise 1 bis 2 bar absolut, regeneriert werden. Die Regeneration mit zwei oder mehr Entspannungsstufen ist beschrieben in den Druckschriften US 4,537, 753, US 4,553, 984, EP 0 159 495, EP 0 202 600, EP 0 190 434 und EP 0 121 109.

Der im Regenerationsschritt b) freigesetzte $CO_2$- und $H_2S$-enthaltende Gasstrom kann beispielsweise in eine Claus-Anlage geleitet werden. In einer Claus-Anlage kann der im Gasstrom enthaltene Schwefelwasserstoff in elementaren Schwefel umgewandelt und der Umwelt dauerhaft entzogen werden. Selbst wenn man jedoch eine $H_2S$-selektive Absorption und die Umwandlung von Schwefelwasserstoff in elementaren Schwefel in einer Claus-Anlage kombiniert, stellt der Rest-Schwefelgehalt im Abgas der Claus-Anlage (Claus-Tailgas) ein Problem dar. Die Rest-Schwefelgehalte des Claus-Tailgases sind in der Regel zu hoch, um das Claus-Tailgas in die Umwelt zu entlassen. Die Erfindung betrifft daher auch vorteilhafte Verschaltungen einer $H_2S$-selektiven Absorption und einer Claus-Anlage, die eine $H_2S$-Entfernung aus dem Claus-Tailgas und/oder eine $H_2S$-Anreicherung im Zulauf zur Claus-Anlage beinhalten.

Mittels einer nachgeschalteten Hydrierungsanlage kann der im Claus-Tailgas enthaltene Schwefel bzw. die sauerstoffhaltigen Schwefelverbindungen und/oder der Schwefelkohlenstoff zu Schwefelwasserstoff hydriert werden. Dieser $H_2S$-haltige Gasstrom kann z. B. wiederum in einem Tailgas-Absorber aufgereinigt werden.

In einer Ausführungsform umfasst das oben beschriebene Verfahren außerdem:

d) einen Schwefelgewinnungsschritt, bei dem man zumindest einen Teilstrom des im Regenerationsschritt b) freigesetzten $CO_2$- und $H_2S$-haltigen Gasstroms in eine Clausanlage leitet, wobei man ein Claus-Tailgas erhält, und das Claus-Tailgas hydriert, wobei man ein hydriertes Claus-Tailgas erhält;

e) einen zweiten Absorptionsschritt, bei dem man das hydrierte Claus-Tailgas mit regeneriertem Absorptionsmittel behandelt, wobei man ein erstes $H_2S$-beladenes Absorptionsmittel erhält;

f) einen Schritt, bei dem man das erste $H_2S$-beladene Absorptionsmittel in den Regenerationsschritt b) und/oder in den Absorptionsschritt a) leitet.

Das hydrierte Claus-Tailgas wird in einen zweiten Absorber geleitet, dessen Kopfstrom $CO_2$-angereichert und $H_2S$-abgereichert ist. Der $CO_2$-angereicherte und $H_2S$-abgereicherte Kopfstrom kann aus dem Verfahren ausgeleitet werden, z. B. einer Verbrennung zugeführt werden. Der Sumpfstrom ist ein erstes $H_2S$-beladenes Absorptionsmittel, das mit dem $CO_2$- und $H_2S$-beladenen Absorptionsmittel vereinigt und in Regenerationsschritt b) geleitet werden kann. Da das erste $H_2S$-beladene Absorptionsmittel in der Regel nicht vollständig beladen ist und daher noch $CO_2$ und/oder $H_2S$ aufnehmen kann, kann man das erste $H_2S$-beladene Absorptionsmittel zur Nutzung der Restkapazität auch ganz oder teilweise in den Absorptionsschritt a) leiten.

In der Regel erfolgt der zweite Absorptionsschritt e) bei einem niedrigeren Druck als der Absorptionsschritt a). Da die $H_2S$-Selektivität des erfindungsgemäß verwendeten Absorptionsmittels bei niedrigeren $H_2S$-Partialdrücken höher ist, erreicht man auf diese Weise eine effektive $H_2S$-Entfernung und eine $H_2S$-Anreicherung im Zulauf zur Claus-Anlage.

In einer weiteren bevorzugten Ausführungsform umfasst das Verfahren außerdem:

d') einen dritten Absorptionsschritt, bei dem man einen Teilstrom eines im Regenerationsschritt b) freigesetzten $CO_2$- und $H_2S$-haltigen Gasstroms mit regeneriertem Absorptionsmittel behandelt, wobei man ein zweites $H_2S$-beladenes Absorptionsmittel erhält;

e') einen Schritt, bei dem man das zweite $H_2S$-beladene Absorptionsmittel in den Regenerationsschritt b) leitet.

Die Ausführungsform umfasst einen Absorptionsschritt, in dem ein Teilstrom des im Regenerationsschritt b) freigesetzten $CO_2$- und $H_2S$-haltigen Gasstroms mit regeneriertem Absorptionsmittel behandelt wird. Der $CO_2$-angereicherte und $H_2S$-abgereicherte Kopfstrom kann aus dem Verfahren ausgeleitet werden, z. B. einer Verbrennung zugeführt werden. Der Sumpfstrom ist ein zweites $H_2S$-beladenes Absorptionsmittel, das mit dem $CO_2$- und $H_2S$-beladenen Absorptionsmittel vereinigt und in den Regenerationsschritt b) geleitet werden kann. Man erreicht auf diese Weise eine $H_2S$-Anreicherung im $CO_2$- und $H_2S$-haltigen Gasstrom, der im Regenerationsschritt b) freigesetzt wird.

In einer besonders bevorzugten Ausführungsform des Verfahrens leitet man einen Teilstrom des $CO_2$- und

$H_2S$-beladenen Absorptionsmittels aus dem Schritt a) in den dritten Absorptionsschritt d'. Die verbleibende Absorptionskapazität des Absorptionsmittels aus dem ersten Absorber kann so genutzt werden.

In einer weiteren bevorzugten Ausführungsform umfasst das vorstehend beschriebene Verfahren außerdem:

f') einen Schwefelgewinnungsschritt, bei dem man einen Teilstrom des in Regenerationsschritt b) freigesetzten $CO_2$- und $H_2S$-haltigen Gasstroms in eine Clausanlage leitet, wobei man ein Claus-Tailgas erhält, und das Claus-Tailgas hydriert, wobei man ein hydriertes Claus-Tailgas erhält;

g') einen zweiten Absorptionsschritt, bei dem man das hydrierte Claus-Tailgas mit regeneriertem Absorptionsmittel behandelt, wobei man ein erstes $H_2S$-beladenes Absorptionsmittel erhält;

h') einen Schritt, bei dem man das erste $H_2S$-beladene Absorptionsmittel in den Regenerationsschritt b) und/oder in den Absorptionsschritt a) leitet.

Das Verfahren umfasst die Behandlung des hydrierten Claus-Tailgases in einem zweiten Absorptionsschritt und die Behandlung eines Teilstroms des im Regenerationsschritt b) freigesetzten $CO_2$- und $H_2S$-haltigen Gasstroms in einem dritten Absorptionsschritt. Man erreicht auf diese Weise eine effektive $H_2S$-Anreicherung im Zulauf zur Claus-Anlage.

In einer bevorzugten Ausführungsform, die mit allen vorstehend beschriebenen Ausführungsformen vorteilhaft kombinierbar ist, umfasst das Verfahren außerdem:
i") einen Rückführschritt, bei dem man einen Teilstrom des im Regenerationsschritt b) freigesetzten $CO_2$- und $H_2S$-haltigen Gasstroms zurückführt und in den Absorptionsschritt a) leitet.

Man erreicht auf diese Weise eine $H_2S$-Anreicherung im $CO_2$- und $H_2S$-haltigen Gasstrom, der im Regenerationsschritt b) freigesetzt wird.

In einer weiteren bevorzugten Ausführungsform erfolgt die Regeneration des $CO_2$- und $H_2S$-beladene Absorptionsmittel zweistufig, wobei in der ersten Stufe ein überwiegend $CO_2$-haltiger Gasstrom freigesetzt wird, der geringe Mengen an $H_2S$ enthält. Der $CO_2$-haltiger Gasstrom wird einem weiteren Absorptionsschritt unterzogen. Der Regenerationsschritt b) umfasst in dieser Ausführungsform:

b1) einen ersten Regenerationsschritt, bei dem man das $CO_2$- und $H_2S$-beladene Absorptionsmittel entspannt, wobei man einen $CO_2$-haltigen Gasstrom und ein teilregeneriertes Absorptionsmittel erhält; und
b2) einen zweiten Regenerationsschritt, bei dem man das teilregenerierte Absorptionsmittel erwärmt und/oder strippt, wobei man das regenerierte Absorptionsmittel erhält.

Das Verfahren umfasst außerdem:

d") einen vierten Absorptionsschritt, bei dem man den $CO_2$-haltigen Gasstrom mit regeneriertem Absorptionsmittel behandelt, wobei man ein drittes $H_2S$-beladenes Absorptionsmittel erhält;

e") einen Schritt, bei dem man das dritte $H_2S$-beladene Absorptionsmittel in den Regenerationsschritt b) leitet.

Die Entfernung einer Hauptmenge des Kohlendioxids aus dem $CO_2$- und $H_2S$-beladenen Absorptionsmittel durch Entspannung reichert das $H_2S$ relativ zum $CO_2$ an und reduziert die Anlagengröße und den Absorptionsmittelumlauf.

In einer besonders bevorzugten Ausführungsform umfasst das Verfahren außerdem:
f") einen Entspannungsschritt, bei dem man das $CO_2$- und $H_2S$-beladene Absorptionsmittel auf einen Druck entspannt, der zwischen dem Druck im Absorptionsschritt a) und dem Druck im ersten Regenerationsschritt b1) liegt, um im Wesentlichen von Kohlendioxid und Schwefelwasserstoff verschiedene gelöste Gasbestandteile aus dem $CO_2$- und $H_2S$-beladene Absorptionsmittel freizusetzen.

Diese Verschaltungsvariante der Anlage erlaubt eine höhere Reinheit der erzeugten $CO_2$-angereicherten sowie $H_2S$-angereicherten Gasströme.

Die Erfindung wird anhand der beigefügten Zeichnungen und der nachfolgenden Beispiele näher veranschaulicht. In den Fig. 1 bis 8 wurden für Elemente gleicher Funktion gleiche Bezugszeichen verwendet. In den Figuren sind zum Verständnis nicht notwendige Anlagenteile, wie Pumpen, der Übersichtlichkeit halber nicht abgebildet.

Fig. 1 ist eine schematische Darstellung einer zur Durchführung des erfindungsgemäßen Verfahrens geeigneten Anlage.

Fig. 2 ist eine schematische Darstellung einer weiteren zur Durchführung des erfindungsgemäßen Verfahrens geeigneten Anlage.

Fig. 3 ist eine schematische Darstellung einer weiteren zur Durchführung des erfindungsgemäßen Verfahrens geeigneten Anlage.

Fig. 4 ist eine schematische Darstellung einer weiteren zur Durchführung des erfindungsgemäßen Verfahrens geeigneten Anlage.

Fig. 5 ist eine schematische Darstellung einer weiteren zur Durchführung des erfindungsgemäßen Verfahrens geeigneten Anlage.

Fig. 6 ist eine schematische Darstellung einer weiteren zur Durchführung des erfindungsgemäßen Verfahrens geeigneten Anlage.

Fig. 7 ist eine schematische Darstellung einer weiteren zur Durchführung des erfindungsgemäßen Verfahrens geeigneten Anlage.

Fig. 8 ist eine schematische Darstellung einer weiteren zur Durchführung des erfindungsgemäßen Verfahrens geeigneten Anlage.

Fig. 9 zeigt die $H_2S$-Selektivität von TBAEE und MDEA in Abhängigkeit von der Beladung bei kleinem $H_2S$-Partialdruck.

Fig. 10 zeigt die $H_2S$-Selektivität von TBAEE, MDEA und einem TBAEE/MDEA-Gemisch in Abhängigkeit von der Beladung bei hohem $H_2S$-Partialdruck.

Fig. 11 zeigt die $H_2S$-Selektivität von MDEA und einem TBAEE/MDEA-Gemisch in Abhängigkeit von der Absorptionsmittel-Umlaufrate bei konstanter Reboiler-Leistung.

Fig. 12 zeigt die $H_2S$-Selektivität von 1,2-Bis(tert-butylamino)ethan (Bis-TBAE) und MDEA in Abhängigkeit von der Beladung bei kleinem $H_2S$-Partialdruck.

Fig. 13 zeigt die $H_2S$-Selektivität von 1,2-Bis(tert-butylamino)ethan (Bis-TBAE) und MDEA in Abhängigkeit von der Beladung bei hohem $H_2S$-Partialdruck.

Gemäß Fig. 1 wird über die Zuleitung Z ein geeignet vorbehandeltes, Schwefelwasserstoff und Kohlendioxid enthaltendes Gas in einem Absorber A1 mit regeneriertem Absorptionsmittel, das über die Absorptionsmittelleitung 1.01 zugeführt wird, im Gegenstrom in Kontakt gebracht. Das Absorptionsmittel entfernt Schwefelwasserstoff und Kohlendioxid durch Absorption aus dem Gas; dabei wird über die Abgasleitung 1.02 ein an Schwefelwasserstoff und Kohlendioxid abgereichertes Reingas gewonnen.

Über die Absorptionsmittelleitung 1.03, den Wärmetauscher 1.04, in dem das $CO_2$- und $H_2S$-beladene Absorptionsmittel mit der Wärme des über die Absorptionsmittelleitung 1.05 geführten, regenerierten Absorptionsmittels aufgeheizt wird, und die Absorptionsmittelleitung 1.06 wird das mit $CO_2$- und $H_2S$-beladene Absorptionsmittel der Desorptionskolonne D zugeleitet und regeneriert.

Zwischen Absorber A1 und Wärmetauscher 1.04 kann ein Entspannungsbehälter vorgesehen sein (in Fig. 1 nicht dargestellt) in dem das $CO_2$- und $H_2S$-beladene Absorptionsmittel auf z. B. 3 bis 15 bar entspannt wird.

Aus dem unteren Teil der Desorptionskolonne D wird das Absorptionsmittel in den Aufkocher 1.07 geführt, wo es erhitzt wird. Der hauptsächlich wasserhaltige Dampf wird in die Desorptionskolonne D zurückgeführt, während das regenerierte Absorptionsmittel über die Absorptionsmittelleitung 1.05, den Wärmetauscher 1.04, in dem das regenerierte Absorptionsmittel das $CO_2$- und $H_2S$-beladene Absorptionsmittel aufheizt und selbst dabei abkühlt, die Absorptionsmittelleitung 1.08, den Kühler 1.09 und die Absorptionsmittelleitung 1.01 dem Absorber A1 wieder zugeführt wird. Anstelle des gezeigten Aufkochers können auch andere Wärmetauschertypen zur Erzeugung des Strippdampfes eingesetzt werden, wie ein Naturumlaufverdampfer, Zwangsumlaufverdampfer, oder Zwangsumlaufentspannungsverdampfer. Bei diesen Verdampfertypen wird ein gemischtphasiger Strom aus regeneriertem Absorptionsmittel und Strippdampf in den Sumpf der Desorptionskolonne zurückgefahren, wo die Phasentrennung zwischen dem Dampf und dem Absorptionsmittel statt. Das regenerierte Absorptionsmittel zum Wärmetauscher 1.04 wird entweder aus dem Umlaufstrom vom Sumpf der Desorptionskolonne zum Verdampfer abgezogen, oder über eine separate Leitung direkt aus dem Sumpf

der Desorptionskolonne zum Wärmetauscher 1.04 geführt.

Das in der Desorptionskolonne D freigesetzte $CO_2$- und $H_2S$-haltige Gas verlässt die Desorptionskolonne D über die Abgasleitung 1.10. Es wird in einen Kondensator mit integrierter Phasentrennung 1.11 geführt, wo es von mitgeführtem Absorptionsmitteldampf getrennt wird. In dieser und allen andern zur Durchführung des erfindungsgemäßen Verfahrens geeigneten Anlagen können Kondensation und Phasentrennung auch getrennt voneinander vorliegen. Anschließend wird eine hauptsächlich aus Wasser bestehende Flüssigkeit über die Absorptionsmittellei-Absorptionsmittelleitung 1.12 in den oberen Bereich der Desorptionskolonne D geführt, und ein $CO_2$- und $H_2S$-haltiges Gas über die Gasleitung 1.13 ausgeführt.

Gemäß Fig. 2 wird über eine Zuleitung Z ein geeignet vorbehandeltes, $CO_2$ und $H_2S$ enthaltendes Gas, bevorzugter Weise Erdgas, in einem Absorber A1 mit regeneriertem Absorptionsmittel, das über die Absorptionsmittelleitung 2.01 zugeführt wird, im Gegenstrom in Kontakt gebracht. Das Absorptionsmittel entfernt $CO_2$ und $H_2S$ durch Absorption aus dem Gas; dabei wird über eine Abgasleitung 2.02 ein $CO_2$- und $H_2S$-abgereichertes Gas gewonnen. Über eine Absorptionsmittelleitung 2.03 wird das $CO_2$- und $H_2S$-beladene Absorptionsmittel in einen Entspannungsbehälter HPF geleitet und entspannt (z. B. von etwa 70 bar auf 3 bis 15 bar, bevorzugt 5 bis 10 bar), wobei die Temperatur im Wesentlichen gleich der Temperatur des beladenen Absorptionsmittels ist. Typischerweise ist die Temperaturdifferenz kleiner als 10 °C, bevorzugt kleiner als 5 °C. Unter diesen Bedingungen werden im Wesentlichen alle im beladenen Absorptionsmittel enthaltenen Kohlenwasserstoffe als Gas freigesetzt und können über die Leitung 2.04 abgetrennt werden.

Über Absorptionsmittelleitung 2.05, 2.07, Wärmetauscher 2.08, in dem das beladene Absorptionsmittel mit der Wärme des über die Absorptionsmittelleitung 2.09 aus dem unteren Bereich des Aufkochers 2.10 ausgeführten, regenerierten Absorptionsmittels aufgeheizt wird, und Absorptionsmittelleitung 2.11 wird das beladene Absorptionsmittel einer Desorptionskolonne D zugeführt, wo es regeneriert wird. Das regenerierte Absorptionsmittel wird in den Aufkocher 2.10 geführt, wo es erhitzt wird. Der hauptsächlich wasserhaltige Dampf wird in die Desorptionskolonne D zurückgeführt, während das regenerierte Absorptionsmittel über die Absorptionsmittelleitung 2.09, den Wärmetauscher 2.08, Absorptionsmittelleitung 2.12, Kühler 2.13, Absorptionsmittelleitung 2.14 abgeführt und in zwei Teilströme 2.01 und 2.15 aufgeteilt und den Absorbern A1 bzw. TGA zugeführt wird. Die relativen Volumenströme der Ströme 2.01 bzw. 2.15 können variiert werden, um die gewünschten Spezifikationen des Abgases auch bei beispielsweise variierendem $H_2S$-Gehalt des zu behandelnden Fluidstroms zu erreichen.

Das in der Desorptionskolonne D gewonnene, $CO_2$ und $H_2S$ enthaltende Gas verlässt die Desorptionskolonne D über die Gasleitung 2.16 und wird im Kondensator mit integrierter Phasentrennung 2.17 von mitgeführtem Dampf getrennt, wonach eine hauptsächlich aus Wasser bestehende Flüssigkeit über die Absorptionsmittelleitung 2.18 in den oberen Bereich der Desorptionskolonne D zurückgeführt wird. Das $CO_2$ und $H_2S$ enthaltende Gas wird über die Gasleitung 2.19 einer Claus-Anlage CL zugeführt, deren Abgas einer Hydrierungsanlage HY zugeführt wird. Das hydrierte Claus-Tailgas wird in den Tailgas-Absorber TGA eingespeist, wo es mit dem über die Absorptionsmittelleitung 2.15 zugeführten, regenerierten Absorptionsmittel im Gegenstrom in Kontakt gebracht wird. Über eine Gasleitung 2.20 wird $CO_2$-angereichertes Gas aus dem Tailgasabsorber TGA abgeführt. Über eine Absorptionsmittelleitung 2.06 wird das $H_2S$-beladene Absorptionsmittel mit dem in Leitung 2.05 geführten beladenen Absorptionsmittel vereinigt und über die Absorptionsmittelleitung 2.07 zur Desorptionskolonne D geleitet.

Die in Fig. 3 schematisch dargestellte Anlage entspricht der Anlage von Fig. 2, wobei jedoch über die Absorptionsmittelleitung 2.06 das $H_2S$-beladene Absorptionsmittel aus dem Tailgasabsorber TGA in den mittleren Bereich des Absorbers A1 eingespeist wird.

Gemäß Fig. 4 wird über eine Zuleitung Z ein geeignet vorbehandeltes, $CO_2$ und $H_2S$ enthaltendes Gas in einem Absorber A1 mit regeneriertem Absorptionsmittel, das über die Absorptionsmittelleitung 4.01 zugeführt wird, im Gegenstrom in Kontakt gebracht. Das Absorptionsmittel entfernt $CO_2$ und $H_2S$ durch Absorption aus dem Gas; dabei wird ein an $CO_2$ und $H_2S$ abgereichertes Gas über die Gasleitung 4.02 abgeführt.

Über eine Absorptionsmittelleitung 4.04, Absorptionsmittelleitung 4.06, Wärmetauscher 4.07, in dem das $CO_2$- und $H_2S$-beladene Absorptionsmittel mit der Wärme des über die Absorptionsmittelleitung 4.08 aus dem unteren Bereich des Aufkochers 4.09 ausgeführten, regenerierten Absorptionsmittels aufgeheizt wird, und Absorptionsmittelleitung 4.10 wird das $CO_2$- und $H_2S$-beladene Absorptionsmittel der Desorptionskolonne D zugeführt, wo es regeneriert wird. Das Absorptionsmittel wird in den Aufkocher 4.09 geführt, wo es erhitzt wird. Der hauptsächlich wasserhaltige Dampf wird in die Desorptionskolonne D zurückgeführt, während das regenerierte Absorptionsmittel über die Absorptionsmittelleitung 4.08, den Wärmetauscher 4.07, die Absorptionsmittelleitung 4.11, den Kühler 4.12, die Absorptionsmittelleitung 4.13 abgeführt wird. Das regenerierte Absorptionsmittel wird in die Teilströme 4.01 bzw. 4.14 aufgeteilt und dem oberen Bereich der Absorber A1 und A2 zugeführt. Die relativen Volumenströme in den Absorptionsmittelleitungen 4.01 und 4.14 können variiert werden, um die gewünschten Spezifikationen des Abgases auch bei variievariierendem $H_2S$-Gehalt zu erreichen.

Das in der Desorptionskolonne D gewonnene, an $CO_2$ und $H_2S$ angereicherte Gas verlässt die Desorptionskolonne D über die Gasleitung 4.15 und wird im Kondensator mit integrierter Phasentrennung 4.16 von mitgeführtem

Dampf getrennt, wonach eine hauptsächlich aus Wasser bestehende Flüssigkeit über die Absorptionsmittelleitung 4.17 in den oberen Bereich der Desorptionskolonne D zurückgeführt wird. Das an $CO_2$ und $H_2S$ angereicherte Gas wird über die Gasleitung 4.18 abgeführt. Ein Teilstrom wird über die Gasleitung 4.19 einer weiteren Behandlung zugeführt, und ein Teilstrom wird über die Gasleitung 4.20 in den unteren Bereich des Absorbers A2 eingespeist.

Im Absorber A2 wird das an $CO_2$ und $H_2S$ angereicherte Gas aus der Leitung 4.20 mit dem über die Absorptionsmittelleitung 4.14 zugeführten, regenerierten Absorptionsmittel im Gegenstrom in Kontakt gebracht. Über eine Gasleitung 4.21 wird das Sauergas-abgereicherte Absorptionsmittel aus dem Absorber A2 abgeführt und aus der Anlage ausgeführt. Über eine Absorptionsmittelleitung 4.05 wird das $H_2S$-beladene Absorptionsmittel aus dem Absorber A2 mit dem in Leitung 4.04 geführten $CO_2$- und $H_2S$-beladenen Absorptionsmittel vereinigt und über Absorptionsmittelleitung 4.06 zur Desorptionskolonne D geleitet.

Die in Fig. 5 schematisch dargestellte Anlage entspricht der Anlage von Fig. 4, wobei jedoch ein Teilstrom des $CO_2$- und $H_2S$-beladenen Absorptionsmittels über die Absorptionsmittelleitung 4.23, Kühler 4.24 und Absorptionsmittelleitung 4.25 in den mittleren Teil des Absorbers A2 geleitet wird.

Gemäß Fig. 6 wird über eine Zuleitung Z ein geeignet vorbehandeltes, $CO_2$ und $H_2S$ enthaltendes Gas in einem Absorber A1 mit im oberen Bereich über die Absorptionsmittelleitung 6.01 zugeführtem regenerierten Absorptionsmittel und dem im mittleren Bereich über die Absorptionsmittelleitung 6.02 zugeführten teilbeladenen Absorptionsmittel im Gegenstrom in Kontakt gebracht. Das Absorptionsmittel entfernt Sauergase durch Absorption aus dem Gas; dabei wird ein Sauergas-abgereichertes Gas über die Gasleitung 6.03 abgeführt und aus der Anlage ausgetragen.

Über Absorptionsmittelleitung 6.05 wird das $CO_2$- und $H_2S$-beladene Absorptionsmittel abgezogen und in die Teilströme 6.06 und 6.07 geteilt. Ein Teil des beladenen Absorptionsmittels wird über die Absorptionsmittelleitung 6.07 in den mittleren Teil des Absorbers A2 eingespeist. Die verbleibende Absorptionska-Absorptionskapazität des Absorptionsmittels aus Absorber A1 kann so genutzt werden.

Der andere Teil des $CO_2$- und $H_2S$-beladenen Absorptionsmittels wird über die Absorptionsmittelleitung 6.06, Absorptionsmittelleitung 6.09, Wärmetauscher 6.10, in dem das mit $CO_2$- und $H_2S$-beladene Absorptionsmittel mit der Wärme des über die Absorptionsmittelleitung 6.11 aus dem unteren Bereich des Aufkochers 6.12 ausgeführten, regenerierten Absorptionsmittels aufgeheizt wird, und Absorptionsmittelleitung 6.13 einer Desorptionskolonne D zugeführt, wo es regeneriert wird. Das regenerierte Absorptionsmittel wird in den Aufkocher 6.12 geführt, wo es erhitzt wird. Der hauptsächlich wasserhaltige Dampf wird in die Desorptionskolonne D zurückgeführt, während das regenerierte Absorptionsmittel über die Absorptionsmittelleitung 6.11, den Wärmetauscher 6.10, Absorptionsmittelleitung 6.14, Kühler 6.15 und Absorptionsmittelleitung 6.16 weitergeführt und in die Teilströme 6.01, 6.17 und 6.18 geteilt. Ein Teil des regenerierten Absorptionsmittels wird über die Absorptionsmittelleitung 6.01 in den oberen Teil des Absorbers A1 geführt, ein anderer Teil des regenerierten Absorptionsmittels wird über die Absorptionsmittelleitung 6.17 in den oberen Teil des Absorbers TGA geführt, und ein weiterer Teil des regenerierten Absorptionsmittels wird über die Absorptionsmittelleitung 6.18 in den oberen Teil des Absorbers A2 geführt. Die relativen Volumenströme in den Absorptionsmittelleitungen 6.01, 6.17 und 6.18 können variiert werden, um die gewünschten Spezifikationen des Abgases auch bei variierendem $H_2S$-Gehalt zu erreichen.

Das in der Desorptionskolonne D gewonnene, $CO_2$ und $H_2S$ enthaltende Gas verlässt die Desorptionskolonne D über die Gasleitung 6.19 und wird im Kondensator mit integrierter Phasentrennung 6.20 von mitgeführtem Dampf getrennt, wonach eine hauptsächlich aus Wasser bestehende Flüssigkeit über die Absorptionsmittelleitung 6.21 in den oberen Bereich der Desorptionskolonne D zurückgeführt wird. Das $CO_2$ und $H_2S$ enthaltende Gas wird zum Teil über die Gasleitung 6.23 in den unteren Bereich des Absorbers A2 eingespeist.

Der andere Teilstrom des $CO_2$ und $H_2S$ enthaltenden Gases wird über die Gasleitung 6.24 einer Claus-Anlage CL zugeführt, deren Abgas in der Hydrierungsanlage HY hydriert wird. Das hydrierte Claus-Tailgas wird in den Tailgasabsorber TGA eingespeist, wo es mit dem über die Absorptionsmittelleitung 6.17 zugeführten, regenerierten Absorptionsmittel im Gegenstrom in Kontakt gebracht wird. Über die Absorptionsmittelleitung 6.02 wird das $H_2S$-beladene Ab-Absorptionsmittel aus dem Tailgasabsorber TGA in den mittleren Teil des Absorbers A1 eingespeist. Die verbleibende Absorptionskapazität des Absorptionsmittels aus Tailgasabsorber TGA kann so genutzt werden. Über eine Gasleitung 6.25 wird das $H_2S$-abgereicherte bzw. $CO_2$-angereicherte Gas aus dem Tailgasabsorber TGA abgeführt, mit dem Gasstrom 6.26 vereinigt und über Gasleitung 6.27 ausgeführt.

Im Absorber A2 wird das $CO_2$ und $H_2S$ enthaltende Gas aus Gasleitung 6.23 mit dem über die Absorptionsmittelleitung 6.18 im oberen Bereich zugeführten, regenerierten Absorptionsmittel und dem im mittleren Bereich über die Absorptionsmittelleitung 6.07 zugeführten $CO_2$- und $H_2S$-beladenen Absorptionsmittel aus Absorber A1 im Gegenstrom in Kontakt gebracht. Über eine Gasleitung 6.26 wird das Sauergas-abgereicherte Absorptionsmittel aus dem Absorber A2 abgeführt. Über eine Absorptionsmittelleitung 6.08 wird ein $H_2S$-beladenes Absorptionsmittel aus dem Absorber A2 mit dem in Leitung 6.06 geführten beladenen Absorptionsmittel vereinigt und über Absorptionsmittelleitung 6.09 zur Desorptionskolonne D weitergeleitet.

Die in Fig. 7 schematisch dargestellte Anlage entspricht der Anlage von Fig. 1, wobei jedoch ein Teilstrom des $CO_2$ und $H_2S$ enthaltenden Gases 1.13 über die Leitung 1.14 zur Zuleitung Z zurückgeführt wird. Die Leitung 1.14 kann

einen Kompressor (nicht dargestellt) enthalten, welcher in Anlagen nötig ist, bei denen der Einlassdruck der Zuleitung Z größer ist als der Auslassdruck des Kondensators mit integrierter Phasentrennung 1.11.

Gemäß Fig. 8 wird über eine Zuleitung Z ein geeignet vorbehandeltes, $CO_2$ und $H_2S$ enthaltendes Gas in einem Absorber A1 mit regeneriertem Absorptionsmittel, das über die Absorptionsmittelleitung 8.01 zugeführt wird, im Gegenstrom in Kontakt gebracht. Das Absorptionsmittel entfernt $CO_2$ und $H_2S$ durch Absorption aus dem Gas; dabei wird über eine Gasleitung 8.02 ein $CO_2$- und $H_2S$-abgereichertes Gas gewonnen. Über eine Absorptionsmittelleitung 8.03 wird das $CO_2$- und $H_2S$-beladene Absorptionsmittel in einen Entspannungsbehälter HPF geleitet und entspannt (z. B. von etwa 70 bar auf 3 bis 15 bar, bevorzugt 5 bis 10 bar), wobei die Temperatur im Wesentlichen gleich der Temperatur des beladenen Absorptionsmittels ist. Typischerweise ist die Temperaturdifferenz kleiner als 10 °C, bevorzugt kleiner als 5 °C. Unter diesen Bedingungen werden im Wesentlichen alle im beladenen Absorptionsmittel enthaltenen Kohlenwasserstoffe als Gas freigesetzt und können über die Leitung 8.04 ausgeführt werden.

Über eine Absorptionsmittelleitung 8.05, einen Wärmetauscher 8.06, in dem das $CO_2$- und $H_2S$-beladene Absorptionsmittel mit der Wärme des über die Absorptionsmittelleitung 8.07 aus dem unteren Bereich des Aufkochers 8.08 ausgeführten, regenerierten Absorptionsmittels aufgeheizt wird, und eine Absorptionsmittelleitung 8.09 wird das beladene Absorptionsmittel in einen Entspannungsbehälter LPF geleitet und entspannt (auf weniger als etwa 5 bar, bevorzugt weniger als etwa 3 bar). Unter diesen Bedingungen werden wesentliche Teile des im beladenen Absorptionsmittel enthaltenen Kohlendioxids als Gas freigesetzt und können über die Gasleitung 8.10 abgetrennt werden, wobei ein teilregeneriertes Absorptionsmittel gewonnen wird. Das $CO_2$-Gas enthält hierbei erhebliche Mengen an $H_2S$, welches abgetrennt werden muss, bevor das $CO_2$ ausgeführt werden kann. Hierfür wird das $CO_2$-Gas über einen Kühler 8.11 und die Gasleitung 8.12 in den Absorber LPA eingespeist, wo es mit dem über die Absorptionsmittelleitung 8.13 zugeführten, regenerierten Absorptionsmittel im Gegenstrom in Kontakt gebracht wird. Dabei wird ein $CO_2$-angereichertes Gas gewonnen, welches über eine Gasleitung 8.14 aus der Anlage geführt wird.

Das aus dem unteren Bereich des Entspannungsbehälters LPF ausgeführte, teilregenerierte Absorptionsmittel und das aus dem unteren Bereich des Absorbers LPA ausgeführte, $H_2S$-beladene Absorptionsmittel wird über die Absorptionsmittelleitungen 8.15 bzw. 8.16 in den oberen Bereich der Desorptionskolonne D eingespeist, wo es regeneriert wird. Das regenerierte Absorptionsmittel wird in den Aufkocher 8.08 geführt, wo es erhitzt wird. Der daraus resultierende, hauptsächlich wasserhaltige Dampf wird in die Desorptionskolonne D zurückgeführt, während das regenerierte Absorptionsmittel über Absorptionsmittelleitung 8.07, Wärmetauscher 8.06, Absorptionsmittelleitung 8.17, Kühler 8.18 und Absorptionsmittelleitung 8.19 abgeführt und in zwei Teilströme 8.01 und 8.13 aufgeteilt und den Absorbern A1 bzw. LPA zugeführt wird.

Das in der Desorptionskolonne D gewonnene, Sauergas-angereicherte Gas verlässt die Desorptionskolonne D über die Gasleitung 8.20 und wird dem Kondensator mit integrierter Phasentrennung 8.21 zugeführt. Im Kondensator mit integrierter Phasentrennung 8.21 wird der Gasstrom von mitgeführtem Dampf getrennt, wonach eine hauptsächlich aus Wasser bestehende Flüssigkeit über die Absorptionsmittelleitung 8.22 in den oberen Bereich der Desorptionskolonne D geführt wird, und ein Sauergas-angereichertes Gas über die Gasleitung 8.23 ausgeführt wird.

Beispiel 1

In einer Pilotanlage wurde die $H_2S$-Selektivität von TBAEE gegenüber MDEA bzw. TBAEE + MDEA bei verschiedenen Absorptionsmittel-Umlaufraten untersucht.

Die Pilotanlage entsprach der Fig. 1. Im Absorber wurde eine strukturierte Packung verwendet. Es herrschte ein Druck von 60 bar. Die Packungshöhe im Absorber betrug 3,2 m bei einem Kolonnendurchmesser von 0,0531 m. Im Desorber wurde eine strukturierte Packung verwendet. Es herrschte ein Druck von 1,8 bar. Die Packungshöhe des Desorbers betrug 6,0 m bei einem Durchmesser von 0,085 m.

Ein Gasgemisch aus 96 Vol.-% $N_2$, 2 Vol.-% $CO_2$ und 2 Vol.-% $H_2S$ wurde mit einem Massenstrom von 47 kg/h und einer Temperatur von 40 °C in den Absorber geleitet. Im Absorber wurde die Absorptionsmittel-Umlaufrate von 30 bis 100 kg/h variiert. Die Temperatur des Absorptionsmittels betrug 50 °C. $H_2S$ wurde auf weniger als 80 ppm entfernt. Die folgende Tabelle zeigt die Ergebnisse dieser Versuche:

| Beispiel | System | Absorptionsmittel-Umlaufrate [kg/h] | Selektivität |
|---|---|---|---|
| 1-1* | TBAEE | 30 | ** |
| 1-2* | TBAEE | 42 | 1,14 |
| 1-3* | TBAEE | 60 | 1,11 |
| 1-4* | MDEA | 60 | 1,35 |

(fortgesetzt)

| Beispiel | System | Absorptionsmittel-Umlaufrate [kg/h] | Selektivität |
|---|---|---|---|
| 1-5 | MDEA + TBAEE | 60 | 1,11 |
| * Vergleichsbeispiele<br>** $H_2S$-Spezifikation nicht erreicht | | | |

Bei der niedrigen Absorptionsmittel-Umlaufrate im Vergleichsbeispiel 1-1 war die Exothermie der Absorption im TBAEE-basierten Absorptionsmittel zu hoch, so dass eine Spezifikation von weniger als 80 ppm $H_2S$ im behandelten Fluidstrom nicht erreicht werden konnte. Bei etwas höherer Umlaufrate (Beispiel 1-2) wird die Trennaufgabe gelöst. Es ist erkennbar, dass die Selektivität von TBAEE bei gleicher Absorptionsmittel-Umlaufrate (Beispiel 1-3) geringer ist als die von MDEA (Vergleichsbeispiel 1-4). Auch die Kombination von MDEA + TBAEE (Beispiel 1-5) weist eine geringere Selektivität auf als reines MDEA.

Beispiel 2

In einer Absorptionseinheit gemäß Beispiel 13 der EP 0 084 943 A2 wurden Absorptionsversuche mit verschiedenen Absorptionsmitteln durchgeführt.

In einem ersten Versuch (als Vergleichsbeispiel) wurde ein Gasgemisch aus 10 Vol.-% $CO_2$ ($CO_2$-Partialdruck 0,1 bar), 1 Vol.-% $H_2S$ ($H_2S$-Partialdruck 0,01 bar) und 89 Vol.-% $N_2$ bei einer Rate von 216 NL/h und bei einer Temperatur von 40 °C durch 100 mL wässriges Absorptionsmittel im Glaszylinder geleitet. Das Absorptionsmittel enthielt 3 M MDEA bzw. 3 M TBAEE. Periodisch wurden Aliquote des Absorptionsmittels abgezogen und der $H_2S$- und $CO_2$-Gehalt bestimmt. Die Ergebnisse sind in Fig. 9 dargestellt. Die $H_2S$-Selektivität ist in Abhängigkeit von der Beladung in Mol($CO_2$ + $H_2S$) pro Mol Amin dargestellt. Es ist erkennbar, dass bei niedrigen Beladungen und niedrigen Partialdrücken sowohl MDEA als auch TBAEE eine hohe Selektivität aufweisen. Mit steigender Beladung nimmt die Selektivität von MDEA ab, während TBAEE weiterhin eine hohe $H_2S$-Selektivität aufweist.

In einem zweiten Versuch wurde ein Gasgemisch aus 90 Vol.-% $CO_2$ ($CO_2$-Partialdruck 0,9 bar), und 10 Vol.-% $H_2S$ ($H_2S$-Partialdruck 0,1 bar), bei einer Rate von 10 NL/h und bei einer Temperatur von 40 °C durch 150 mL wässriges Absorptionsmittel im Glaszylinder geleitet. Das Absorptionsmittel enthielt 1,9 M MDEA, 1,9 M TBAEE bzw. 1,4 M MDEA + 0,5 M TBAEE. Periodisch wurden Aliquote des Absorptionsmittels abgezogen und der $H_2S$- und $CO_2$-Gehalt bestimmt. Die Ergebnisse sind in Fig. 10 dargestellt. Die $H_2S$-Selektivität ist in Abhängigkeit von der Beladung in Mol($CO_2$ + $H_2S$) pro Mol Amin dargestellt. Es zeigte sich, dass bei den größeren Partialdrücken und der höheren Absorptionsmittel-Umlaufrate die $H_2S$-Selektivität mit zunehmender Beladung bei allen Absorptionsmitteln ansteigt, bis ein Plateau erreicht ist. Die $H_2S$-Selektivität von MDEA ist höher als die von TBAEE, wobei die $H_2S$-Selektivität von TBAEE + MDEA zwischen MDEA und TBAEE liegt.

Beispiel 3

Es wurden Absorptionsversuche in einer Pilotanlage durchgeführt. Die Pilotanlage war wie in Beispiel 1 aufgebaut.

Die $H_2S$-Selektivität eines wässrigen Absorptionsmittels, das 40 Gew.-% MDEA enthielt, sowie eines wässrigen Absorptionsmittels, das 30 Gew.-% MDEA und 15 Gew.-% TBAEE enthielt, wurde in Erdgas bei verschiedenen Absorptions-Umlaufraten untersucht. Es lagen Konzentrationen von 5% $CO_2$ und 2% $H_2S$ im Erdgasstrom vor. $H_2S$ wurde auf weniger als 10 ppm entfernt. Es herrschte ein Druck von 60 bar. Die zur Regeneration des Absorptionsmittels benötigte Energie (Reboiler-Leistung) wurde konstant gehalten und die resultierende $H_2S$-Selektivität der Absorptionsmittel untersucht. Fig. 11 zeigt die Messdaten.

Es ist erkennbar, dass die $H_2S$-Selektivität bei einer niedrigen Absorptionsmittel-Umlaufrate höher ist. Hier liegen auch die Selektivitäten der Absorptionsmittel mit MDEA bzw. MDEA + TBAEE noch nah beieinander, wobei die Selektivität des MDEA + TBAEE-Gemischs stets niedriger ist. In beiden Fällen nimmt die Selektivität ab, wenn die Absorptionsmittel-Umlaufrate erhöht wird. Allerdings ist ab ca. 50 kg/h die Selektivität des MDEA-Absorptionsmittels relativ konstant, während die Selektivität des TBAEE + MDEA-Gemischs weiter abnimmt. Je höher also die Absorptionsmittel-Umlaufrate, desto günstiger ist die Verwendung von TBAEE mit MDEA gegenüber reinem MDEA, wenn neben einem hohen $H_2S$-Abtrennungsgrad auch eine hohe Kohlendioxid-Coabsorption unter Erhalt definierter Mindestmengen erzielt werden soll.

Beispiel 4

In einer Absorptionseinheit gemäß Beispiel 13 der EP 0 084 943 A2 wurden Absorptionsversuche mit verschiedenen Absorptionsmitteln durchgeführt.

In einem ersten Versuch wurde ein Gasgemisch aus 10 Vol.-% $CO_2$ ($CO_2$-Partialdruck 0,1 bar), 1 Vol.-% $H_2S$ ($H_2S$-Partialdruck 0,01 bar) und 89 Vol.-% $N_2$ bei einer Rate von 216 NL/h und bei einer Temperatur von 40 °C durch 100 mL wässriges Absorptionsmittel im Glaszylinder geleitet. Das Absorptionsmittel enthielt 0,64 M MDEA bzw. 0,64 M 1,2-Bis(tert-butylamino)ethan (Bis-TBAE). Periodisch wurden Aliquote des Absorptionsmittels abgezogen und der $H_2S$- und $CO_2$-Gehalt bestimmt. Die Ergebnisse sind in Fig. 12 dargestellt. Die $H_2S$-Selektivität ist in Abhängigkeit von der Beladung in Mol($CO_2$ + $H_2S$) pro Mol Amin dargestellt. Es ist erkennbar, dass bei niedrigen Beladungen und niedrigen Partialdrücken MDEA und Bis-TBAE eine relativ ähnliche Selektivität aufweisen. Mit steigender Beladung nimmt die Selektivität von MDEA deutlich rascher ab, als die $H_2S$-Selektivität von Bis-TBAE.

In einem zweiten Versuch wurde ein Gasgemisch aus 90 Vol.-% $CO_2$ ($CO_2$-Partialdruck 0,9 bar), und 10 Vol.-% $H_2S$ ($H_2S$-Partialdruck 0,1 bar), bei einer Rate von 10 NL/h und bei einer Temperatur von 40 °C durch 150 mL wässriges Absorptionsmittel im Glaszylinder geleitet. Das Absorptionsmittel enthielt 0,64 M MDEA bzw. 0,64 M 1,2-Bis(tert-butylamino)ethan (Bis-TBAE). Periodisch wurden Aliquote des Absorptionsmittels abgezogen und der $H_2S$- und $CO_2$-Gehalt bestimmt. Die Ergebnisse sind in Fig. 13 dargestellt. Die $H_2S$-Selektivität ist in Abhängigkeit von der Beladung in Mol($CO_2$ + $H_2S$) pro Mol Amin dargestellt. Es zeigte sich, dass bei den größeren Partialdrücken und der höheren Absorptionsmittel-Umlaufrate die $H_2S$-Selektivität von MDEA höher ist als die von Bis-TBAE.

## Patentansprüche

1. Verfahren zur Entfernung von Schwefelwasserstoff und Kohlendioxid aus einem unter Gasen ausgewählten Fluidstrom, umfassend

   a) einen Absorptionsschritt, bei dem man den Fluidstrom mit einem Absorptionsmittel in Kontakt bringt, das eine wässrige Lösung von (i) 2-(2-tert-Butylaminoethoxy)ethanol und (ii) Methyldiethanolamin umfasst, wobei das molare Verhältnis von (i) zu (ii) im Bereich von 0,1 bis 0,9 liegt;
   wobei im Fluidstrom ein Schwefelwasserstoff-Partialdruck von mindestens 0,1 bar und ein Kohlendioxid-Partialdruck von mindestens 1 bar vorliegt;
   wobei der Fluidstrom einen Gesamtdruck von mindestens 20 bar aufweist; wobei mindestens 90% des Schwefelwasserstoffs aus dem Fluidstrom entfernt werden und die Selektivität für Schwefelwasserstoff gegenüber Kohlendioxid nicht größer als 8 ist,
   wobei man ein $CO_2$- und $H_2S$-beladenes Absorptionsmittel und einen behandelten Fluidstrom erhält;
   wobei unter Selektivität für Schwefelwasserstoff gegenüber Kohlendioxid der Wert des Quotienten (S) verstanden wird

$$\frac{\dfrac{y(H_2S)_{feed} - y(H_2S)_{treat}}{y(H_2S)_{feed}}}{\dfrac{y(CO_2)_{feed} - y(CO_2)_{treat}}{y(CO_2)_{feed}}} \quad (S),$$

   worin $y(H_2S)_{feed}$ für den Stoffmengenanteil (mol/mol) von $H_2S$ im Fluidstrom, $y(H_2S)_{treat}$ für den Stoffmengenanteil im behandelten Fluidstrom, $y(CO_2)_{feed}$ für den Stoffmengenanteil von $CO_2$ im Fluidstrom und $y(CO_2)_{treat}$ für den Stoffmengenanteil von $CO_2$ im behandelten Fluidstrom steht;
   b) einen Regenerationsschritt, bei dem man zumindest einen Teilstrom des $CO_2$- und $H_2S$-beladenen Absorptionsmittels regeneriert und man ein regeneriertes Absorptionsmittel erhält; und
   c) einen Rückführschritt, bei dem man zumindest einen Teilstrom des regenerierten Absorptionsmittels in den Absorptionsschritt a) zurückführt.

2. Verfahren nach Anspruch 1, wobei die Gesamtkonzentration von (i) und (ii) in der wässrigen Lösung 10 bis 60 Gew.-% beträgt.

3. Verfahren nach einem der vorhergehenden Ansprüche, wobei das Absorptionsmittel kein sterisch ungehindertes primäres oder sekundäres Amin enthält.

4. Verfahren nach einem der vorhergehenden Ansprüche, wobei das Absorptionsmittel wenigstens ein organisches Lösungsmittel umfasst.

5. Verfahren nach einem der vorhergehenden Ansprüche, wobei der Fluidstrom Kohlenwasserstoffe enthält.

6. Verfahren nach einem der vorhergehenden Ansprüche, wobei man das $CO_2$- und $H_2S$-beladene Absorptionsmittel im Regenerationsschritt b) auf eine $H_2S$-Beladung regeneriert, die einer Gleichgewichtsbeladung für einen $H_2S$-Gehalt von weniger als 90% des $H_2S$-Gehalts des behandelten Fluidstroms entspricht.

7. Verfahren nach einem der vorhergehenden Ansprüche, wobei die kumulierte $CO_2$- und $H_2S$-Beladung des $CO_2$- und $H_2S$-beladenen Absorptionsmittels wenigstens 0,25 mol/mol und die kumulierte $CO_2$- und $H_2S$-Beladung des regenerierten Absorptionsmittels weniger als 0,20 mol/mol beträgt.

8. Verfahren nach einem der vorhergehenden Ansprüche, wobei der Regenerationsschritt b) wenigstens eine der Maßnahmen Erwärmung, Entspannung und Strippen mit einem inerten Fluid umfasst.

9. Verfahren nach einem der vorhergehenden Ansprüche, außerdem umfassend:

d) einen Schwefelgewinnungsschritt, bei dem man zumindest einen Teilstrom des im Regenerationsschritt b) freigesetzten $CO_2$- und $H_2S$-haltigen Gasstroms in eine Clausanlage leitet, wobei man ein Claus-Tailgas erhält, und das Claus-Tailgas hydriert, wobei man ein hydriertes Claus-Tailgas erhält;
e) einen zweiten Absorptionsschritt, bei dem man das hydrierte Claus-Tailgas mit regeneriertem Absorptionsmittel behandelt, wobei man ein erstes $H_2S$-beladenes Absorptionsmittel erhält;
f) einen Schritt, bei dem man das erste $H_2S$-beladene Absorptionsmittel in den Regenerationsschritt b) und/oder in den Absorptionsschritt a) leitet.

10. Verfahren nach Anspruch 9, wobei der zweite Absorptionsschritt e) bei einem niedrigeren Druck erfolgt als der Absorptionsschritt a).

11. Verfahren nach einem der Ansprüche 1 bis 8, außerdem umfassend:

d') einen dritten Absorptionsschritt, bei dem man einen Teilstrom eines im Regenerationsschritt b) freigesetzten $CO_2$- und $H_2S$-haltigen Gasstroms mit regeneriertem Absorptionsmittel behandelt, wobei man ein zweites $H_2S$-beladenes Absorptionsmittel erhält;
e') einen Schritt, bei dem man das zweite $H_2S$-beladene Absorptionsmittel in den Regenerationsschritt b) leitet.

12. Verfahren nach Anspruch 11, wobei man einen Teilstrom des $CO_2$- und $H_2S$-beladenen Absorptionsmittels in den dritten Absorptionsschritt d' leitet.

13. Verfahren nach Anspruch 11 oder 12, außerdem umfassend:

f') einen Schwefelgewinnungsschritt, bei dem man einen Teilstrom des in Regenerationsschritt b) freigesetzten $CO_2$- und $H_2S$-haltigen Gasstroms in eine Clausanlage leitet, wobei man ein Claus-Tailgas erhält, und das Claus-Tailgas hydriert, wobei man ein hydriertes Claus-Tailgas erhält;
g') einen zweiten Absorptionsschritt, bei dem man das hydrierte Claus-Tailgas mit regeneriertem Absorptionsmittel behandelt, wobei man ein erstes $H_2S$-beladenes Absorptionsmittel erhält;
h') einen Schritt, bei dem man das erste $H_2S$-beladene Absorptionsmittel in den Regenerationsschritt b) und/oder in den Absorptionsschritt a) leitet.

14. Verfahren nach einem der vorhergehenden Ansprüche, außerdem umfassend
i") einen Rückführschritt, bei dem man einen Teilstrom des im Regenerationsschritt b) freigesetzten $CO_2$- und $H_2S$-haltigen Gasstroms zurückführt und in den Absorptionsschritt a) leitet.

15. Verfahren nach einem der Ansprüche 1 bis 8, wobei der Regenerationsschritt b) umfasst:

b1) einen ersten Regenerationsschritt, bei dem man das $CO_2$- und $H_2S$-beladene Absorptionsmittel entspannt, wobei man einen $CO_2$-haltigen Gasstrom und ein teilregeneriertes Absorptionsmittel erhält; und
b2) einen zweiten Regenerationsschritt, bei dem man das teilregenerierte Absorptionsmittel erwärmt und/oder strippt, wobei man das regenerierte Absorptionsmittel erhält;

und das Verfahren außerdem umfasst:

d") einen vierten Absorptionsschritt, bei dem man den $CO_2$-haltigen Gasstrom mit regeneriertem Absorptionsmittel behandelt, wobei man ein drittes $H_2S$-beladenes Absorptionsmittel erhält;
e") einen Schritt, bei dem man das dritte $H_2S$-beladene Absorptionsmittel in den Regenerationsschritt b) leitet.

**16.** Verfahren nach Anspruch 15, außerdem umfassend:
f") einen Entspannungsschritt, bei dem man das $CO_2$- und $H_2S$-beladene Absorptionsmittel auf einen Druck entspannt, der zwischen dem Druck im Absorptionsschritt a) und dem Druck im ersten Regenerationsschritt b1) liegt, um im Wesentlichen von Kohlendioxid und Schwefelwasserstoff verschiedene gelöste Gasbestandteile aus dem $CO_2$- und $H_2S$-beladene Absorptionsmittel freizusetzen.

## Claims

**1.** A process for removing hydrogen sulfide and carbon dioxide from a fluid stream selected from gases, comprising

a) an absorption step in which the fluid stream is contacted with an absorbent comprising an aqueous solution of (i) 2-(2-tert-butylamino-ethoxy)ethanol and (ii) methyldiethanolamine, where the molar ratio of (i) to (ii) is in the range from 0.1 to 0.9;
wherein there is a partial hydrogen sulfide pressure of at least 0.1 bar and a partial carbon dioxide pressure of at least 1 bar in the fluid stream;
wherein the fluid stream has a total pressure of at least 20 bar;
wherein at least 90% of the hydrogen sulfide is removed from the fluid stream and selectivity for hydrogen sulfide over carbon dioxide is not greater than 8, wherein a $CO_2$- and $H_2S$-laden absorbent and a treated fluid stream are obtained;
wherein the selectivity for hydrogen sulfide over carbon dioxide is understood to mean the value of the quotient (S)

$$\frac{\frac{y(H_2S)_{feed} - y(H_2S)_{treat}}{y(H_2S)_{feed}}}{\frac{y(CO_2)_{feed} - y(CO_2)_{treat}}{y(CO_2)_{feed}}} \quad (S),$$

in which $y(H_2S)_{feed}$ is the molar proportion (mol/mol) of $H_2S$ in the fluid stream, $y(H_2S)_{treat}$ is the molar proportion in the treated fluid stream, $y(CO_2)_{feed}$ is the molar proportion of $CO_2$ in the fluid stream and $y(CO_2)_{treat}$ is the molar proportion of $CO_2$ in the treated fluid stream;
b) a regeneration step in which at least a substream of the $CO_2$- and $H_2S$-laden absorbent is regenerated and a regenerated absorbent is obtained; and
c) a recycling step in which at least a substream of the regenerated absorbent is recycled into the absorption step a).

**2.** The process according to claim 1, wherein the total concentration of (i) and (ii) in the aqueous solution is 10% to 60% by weight.

**3.** The process according to either of the preceding claims, wherein the absorbent does not comprise any sterically unhindered primary or secondary amine.

**4.** The process according to any of the preceding claims, wherein the absorbent comprises at least one organic solvent.

**5.** The process according to any of the preceding claims, wherein the fluid stream comprises hydrocarbons.

**6.** The process according to any of the preceding claims, wherein the $CO_2$- and $H_2S$-laden absorbent is regenerated in the regeneration step b) to an $H_2S$ loading corresponding to an equilibrium loading for an $H_2S$ content of less than 90% of the $H_2S$ content of the treated fluid stream.

**7.** The process according to any of the preceding claims, wherein the cumulated $CO_2$ and $H_2S$ loading of the $CO_2$- and $H_2S$-laden absorbent is at least 0.25 mol/mol and the cumulated $CO_2$ and $H_2S$ loading of the regenerated absorbent is less than 0.20 mol/mol.

**8.** The process according to any of the preceding claims, wherein the regeneration step b) comprises at least one of the measures of heating, decompressing and stripping with an inert fluid.

**9.** The process according to any of the preceding claims, also comprising:

d) a sulfur recovery step in which at least a substream of the $CO_2$- and $H_2S$-containing gas stream released in the regeneration step b) is passed into a Claus plant to obtain a Claus tail gas, and the Claus tail gas is hydrogenated to obtain a hydrogenated Claus tail gas;
e) a second absorption step in which the hydrogenated Claus tail gas is treated with regenerated absorbent to obtain a first $H_2S$-laden absorbent;
f) a step in which the first $H_2S$-laden absorbent is passed into the regeneration step b) and/or into the absorption step a).

**10.** The process according to claim 9, wherein the second absorption step e) is effected at a lower pressure than the absorption step a).

**11.** The process according to any of claims 1 to 8, also comprising:

d') a third absorption step in which a substream of a $CO_2$- and $H_2S$-containing gas stream released in the regeneration step b) is treated with regenerated absorbent to obtain a second $H_2S$-laden absorbent;
e') a step in which the second $H_2S$-laden absorbent is passed into the regeneration step b).

**12.** The process according to claim 11, wherein a substream of the $CO_2$- and $H_2S$-laden absorbent is passed into the third absorption step d'.

**13.** The process according to claim 11 or 12, also comprising:

f') a sulfur recovery step in which a substream of the $CO_2$- and $H_2S$-containing gas stream released in regeneration step b) is passed into a Claus plant to obtain a Claus tail gas, and the Claus tail gas is hydrogenated to obtain a hydrogenated Claus tail gas;
g') a second absorption step in which the hydrogenated Claus tail gas is treated with regenerated absorbent to obtain a first $H_2S$-laden absorbent;
h') a step in which the first $H_2S$-laden absorbent is passed into the regeneration step b) and/or into the absorption step a).

**14.** The process according to any of the preceding claims, also comprising
i") a recycling step in which a substream of the $CO_2$- and $H_2S$-containing gas stream released in the regeneration step b) is recycled and passed into the absorption step a).

**15.** The process according to any of claims 1 to 8, wherein the regeneration step b) comprises:

b1) a first regeneration step in which the $CO_2$- and $H_2S$-laden absorbent is decompressed to obtain a $CO_2$-containing gas stream and a partly regenerated absorbent; and
b2) a second regeneration step in which the partly regenerated absorbent is heated and/or stripped to obtain the regenerated absorbent;

and the process also comprises:

d") a fourth absorption step in which the $CO_2$-containing gas stream is treated with regenerated absorbent to obtain a third $H_2S$-laden absorbent;
e") a step in which the third $H_2S$-laden absorbent is passed into the regeneration step b).

**16.** The process according to claim 15, also comprising:
f") a decompression step in which the $CO_2$- and $H_2S$-laden absorbent is decompressed to a pressure between the pressure in the absorption step a) and the pressure in the first regeneration step b1), in order to release essentially dissolved gas constituents other than carbon dioxide and hydrogen sulfide from the $CO_2$- and $H_2S$-laden absorbent.

## Revendications

**1.** Procédé pour l'élimination d'acide sulfhydrique et de dioxyde de carbone d'un flux fluidique choisi parmi les gaz, comprenant

a) une étape d'absorption, dans laquelle on met le flux fluidique en contact avec un agent d'absorption qui comprend une solution aqueuse de (i) 2-(2-tert-butylaminoéthoxy)éthanol et de (ii) méthyldiéthanolamine, le rapport molaire de (i) à (ii) étant situé dans la plage de 0,1 à 0,9 ;
le flux fluidique présentant une pression partielle d'acide sulfhydrique d'au moins 0,1 bar et une pression partielle de dioxyde de carbone d'au moins 1 bar ;
le flux fluidique présentant une pression totale d'au moins 20 bars ;
au moins 90% de l'acide sulfhydrique étant éliminés du flux fluidique et la sélectivité pour l'acide sulfhydrique par rapport au dioxyde de carbone n'étant pas supérieure à 8,
un agent d'absorption chargé de $CO_2$ et de $H_2S$ ainsi qu'un flux fluidique traité étant obtenus ;
la sélectivité pour l'acide sulfhydrique par rapport au dioxyde de carbone signifiant la valeur du quotient (S)

$$\frac{\frac{y(H_2S)_{feed} - y(H_2S)_{treat}}{y(H_2S)_{feed}}}{\frac{y(CO_2)_{feed} - y(CO_2)_{treat}}{y(CO_2)_{feed}}} \quad (S),$$

où y $(H_2S)_{feed}$ représente la proportion de quantité de matière (mole/mole) de $H_2S$ dans le flux fluidique, y $(H_2S)_{treat}$ représente la proportion de quantité de matière dans le flux fluidique traité, $y(CO_2)_{feed}$ représente la proportion de quantité de matière de $CO_2$ dans le flux fluidique et $y(CO_2)_{treat}$ représente la proportion de quantité de matière de $CO_2$ dans le flux fluidique traité ;
b) une étape de régénération, dans laquelle on régénère au moins un flux partiel de l'agent d'absorption chargé de $CO_2$ et de $H_2S$ et on obtient un agent d'absorption régénéré ; et
c) une étape de recyclage, dans laquelle on recycle au moins un flux partiel de l'agent d'absorption régénéré dans l'étape d'absorption a).

**2.** Procédé selon la revendication 1, la concentration totale en (i) et en (ii) dans la solution aqueuse étant de 10 à 60% en poids.

**3.** Procédé selon l'une quelconque des revendications précédentes, l'agent d'absorption ne contenant pas d'amine primaire ou secondaire stériquement non encombrée.

**4.** Procédé selon l'une quelconque des revendications précédentes, l'agent d'absorption comprenant au moins un solvant organique.

**5.** Procédé selon l'une quelconque des revendications précédentes, le flux fluidique contenant des hydrocarbures.

**6.** Procédé selon l'une quelconque des revendications précédentes, l'agent d'absorption chargé de $CO_2$ et de $H_2S$ étant régénéré dans l'étape de régénération b) à une charge de $H_2S$ qui correspond à une charge d'équilibre pour

une teneur en $H_2S$ inférieure à 90% de la teneur en $H_2S$ du flux fluidique traité.

**7.** Procédé selon l'une quelconque des revendications précédentes, la charge cumulée de $CO_2$ et de $H_2S$ de l'agent d'absorption chargé de $CO_2$ et de $H_2S$ étant au moins 0,25 mole/mole et la charge cumulée de $CO_2$ et de $H_2S$ de l'agent d'absorption régénéré étant inférieure à 0,20 mole/mole.

**8.** Procédé selon l'une quelconque des revendications précédentes, l'étape de régénération b) comprenant au moins l'une des mesures chauffage, détente et extraction à l'aide d'un fluide inerte.

**9.** Procédé selon l'une quelconque des revendications précédentes, comprenant en outre :

d) une étape de production de soufre, dans laquelle on guide au moins un flux partiel du flux gazeux contenant $CO_2$ et $H_2S$ libéré dans l'étape de régénération b) dans une installation de Claus, un gaz résiduaire de Claus étant obtenu et le gaz résiduaire de Claus étant hydrogéné, un gaz résiduaire hydrogéné de Claus étant obtenu ;
e) une deuxième étape d'absorption, dans laquelle on traite le gaz résiduaire hydrogéné de Claus avec de l'agent d'absorption régénéré, un premier agent d'absorption chargé de $H_2S$ étant obtenu ;
f) une étape, dans laquelle on guide le premier agent d'absorption chargé de $H_2S$ dans l'étape de régénération b) et/ou dans l'étape d'absorption a).

**10.** Procédé selon la revendication 9, la deuxième étape d'absorption e) ayant lieu à une pression inférieure à celle de l'étape d'absorption a).

**11.** Procédé selon l'une quelconque des revendications 1 à 8, comprenant en outre :

d') une troisième étape d'absorption, dans laquelle on traite un flux partiel d'un flux gazeux contenant $CO_2$ et $H_2S$ libéré dans l'étape de régénération b) avec de l'agent d'absorption régénéré, un deuxième agent d'absorption chargé de $H_2S$ étant obtenu ;
e') une étape, dans laquelle on guide le deuxième agent d'absorption chargé de $H_2S$ dans l'étape de régénération b) .

**12.** Procédé selon la revendication 11, un flux partiel de l'agent d'absorption chargé de $CO_2$ et de $H_2S$ étant guidé dans la troisième étape d'absorption d').

**13.** Procédé selon la revendication 11 ou 12, comprenant en outre :

f') une étape de production de soufre, dans laquelle on guide un flux partiel du flux gazeux contenant $CO_2$ et $H_2S$ libéré dans l'étape de régénération b) dans une installation de Claus, un gaz résiduaire de Claus étant obtenu et le gaz résiduaire de Claus étant hydrogéné, un gaz résiduaire hydrogéné de Claus étant obtenu ;
g') une deuxième étape d'absorption, dans laquelle on traite le gaz résiduaire hydrogéné de Claus avec de l'agent d'absorption régénéré, un premier agent d'absorption chargé de $H_2S$ étant obtenu ;
h') une étape, dans laquelle on guide le premier agent d'absorption chargé de $H_2S$ dans l'étape de régénération b) et/ou dans l'étape d'absorption a).

**14.** Procédé selon l'une quelconque des revendications précédentes, comprenant en outre :
i") une étape de recyclage, dans laquelle on recycle un flux partiel du flux gazeux contenant $CO_2$ et $H_2S$ libéré dans l'étape de régénération b) et on le guide dans l'étape d'absorption a).

**15.** Procédé selon l'une quelconque des revendications 1 à 8, l'étape de régénération b) comprenant :

b1) une première étape de régénération, dans laquelle on détend l'agent d'absorption chargé de $CO_2$ et de $H_2S$, un flux gazeux contenant $CO_2$ et un agent d'absorption partiellement régénéré étant obtenus ; et
b2) une deuxième étape de régénération, dans laquelle on chauffe et/ou extrait l'agent d'absorption partiellement régénéré, l'agent d'absorption régénéré étant obtenu ;

et le procédé comprenant en outre :

d") une quatrième étape d'absorption, dans laquelle on traite le flux gazeux contenant $CO_2$ avec de l'agent d'absorption régénéré, un troisième agent d'absorption chargé de $H_2S$ étant obtenu ;

e") une étape, dans laquelle on guide le troisième agent d'absorption chargé de $H_2S$ dans l'étape de régénération b) .

**16.** Procédé selon la revendication 15, comprenant en outre :
f") une étape de détente, dans laquelle on détend l'agent d'absorption chargé de $CO_2$ et de $H_2S$ à une pression, qui se situe entre la pression dans l'étape d'absorption a) et la pression dans la première étape de régénération b1), pour libérer essentiellement des constituants gazeux dissous, différents du dioxyde de carbone et de l'acide sulfhydrique, de l'agent d'absorption chargé de $CO_2$ et de $H_2S$.

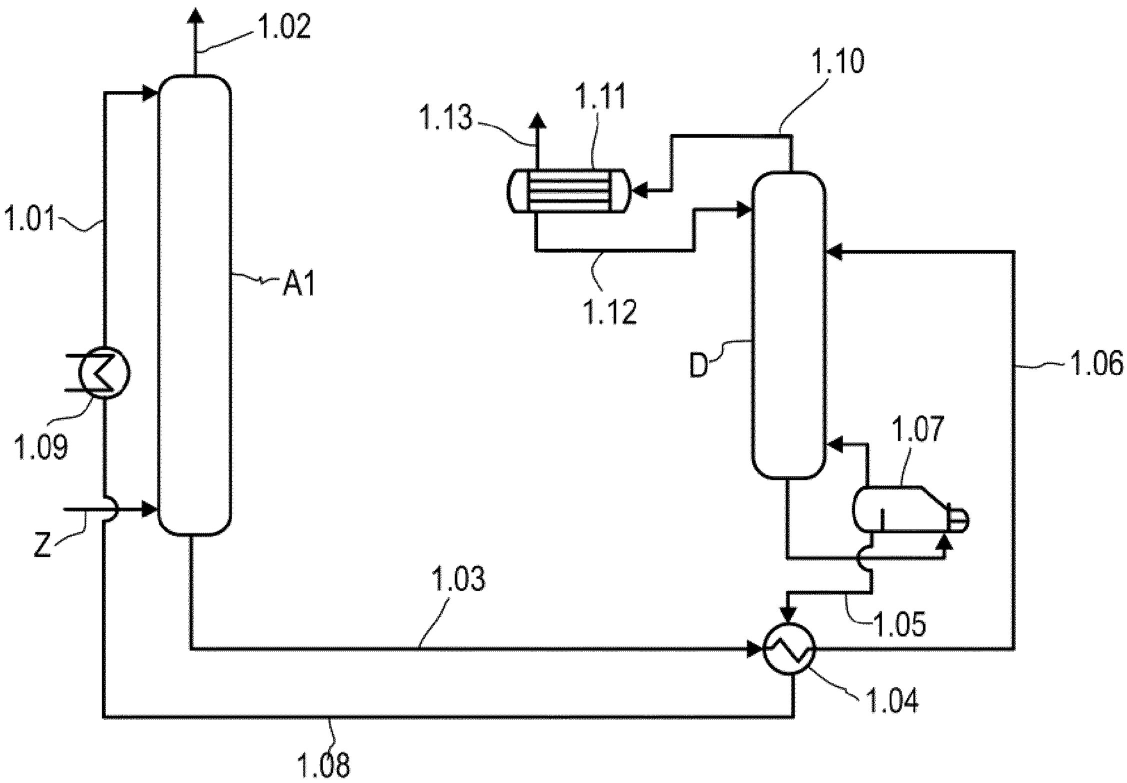


Fig. 1

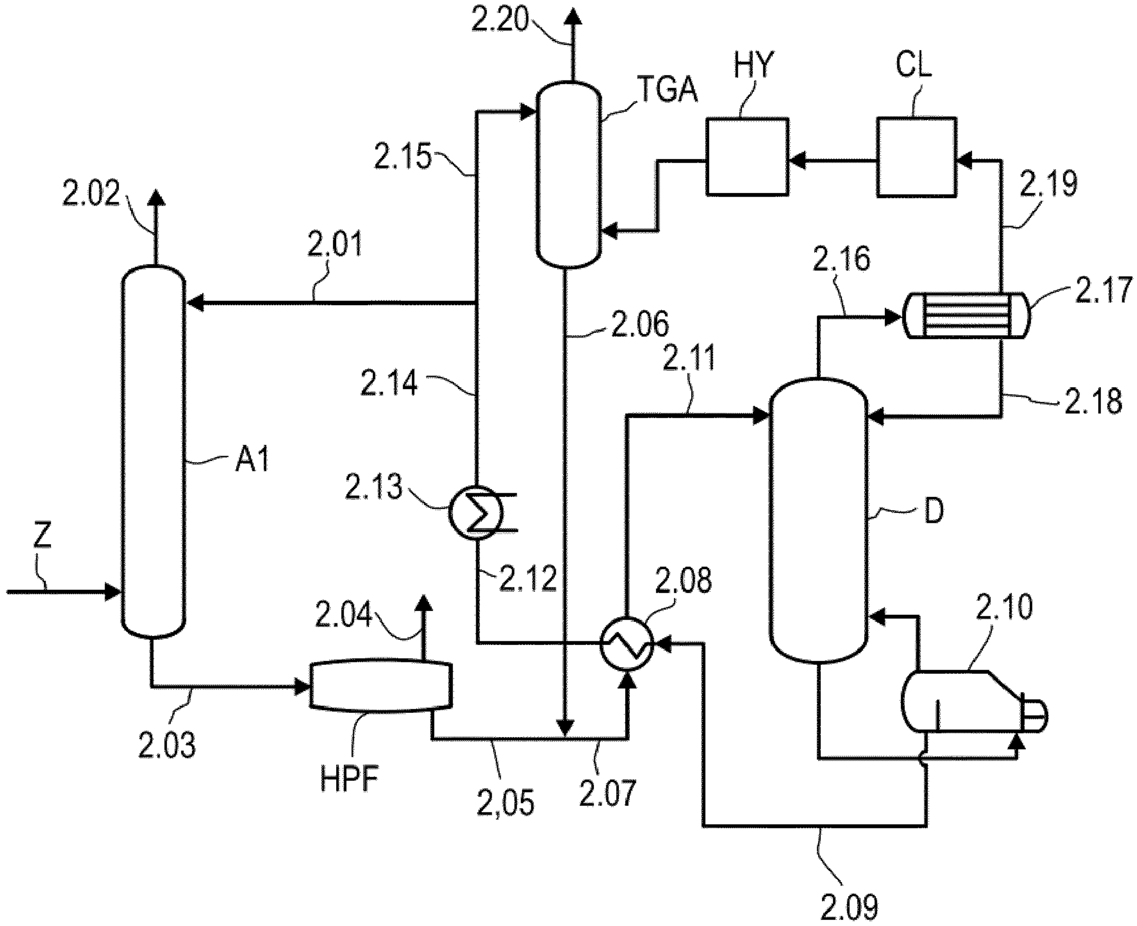
2.20
TGA
HY
CL
2.15
2.19
2.02
2.01
2.16
2.17
2.06
2.11
2.14
2.18
A1
2.13
D
Z
2.12
2.08
2.10
2.04
2.03
HPF
2,05
2.07
2.09

Fig. 2

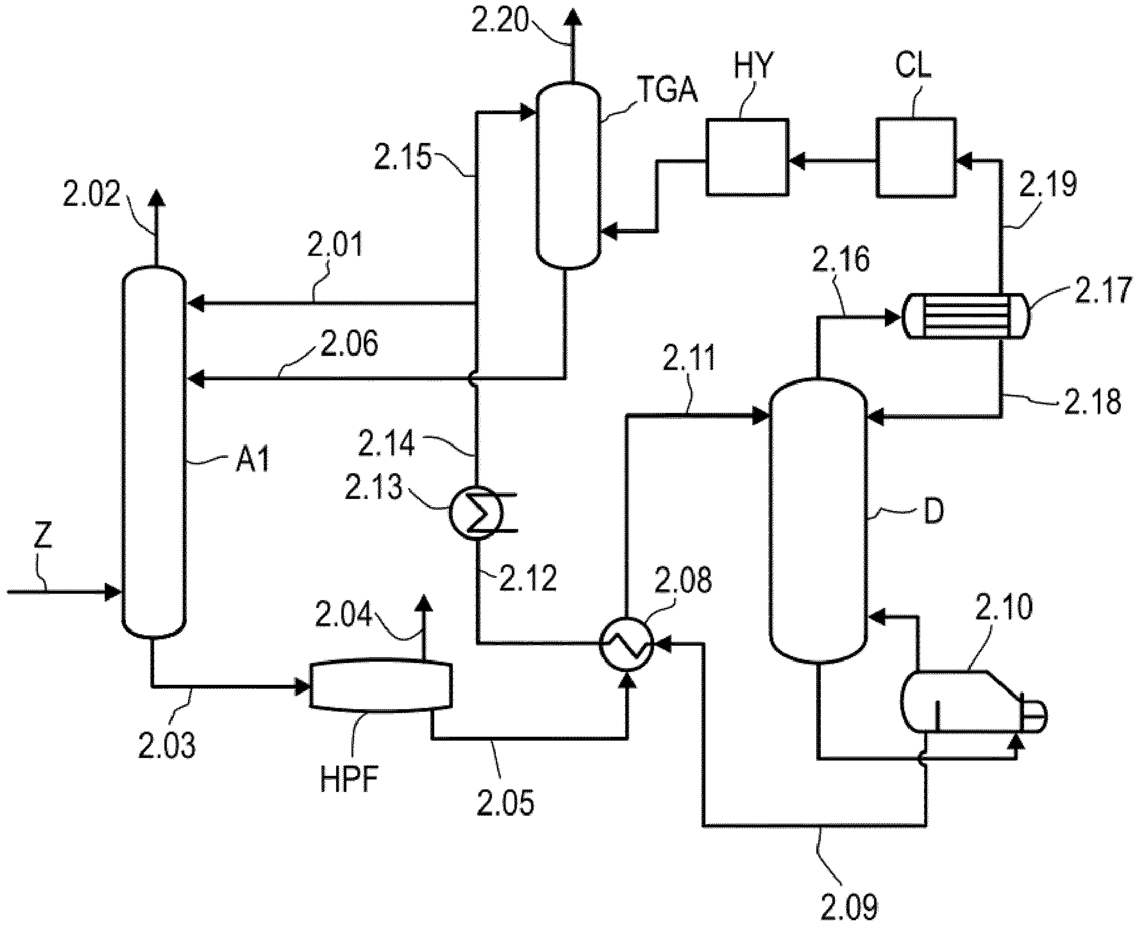
2.20
TGA
HY
CL
2.15
2.19
2.02
2.01
2.16
2.17
2.06
2.11
2.18
2.14
A1
2.13
D
Z
2.12
2.08
2.10
2.04
2.03
HPF
2.05
2.09

Fig. 3

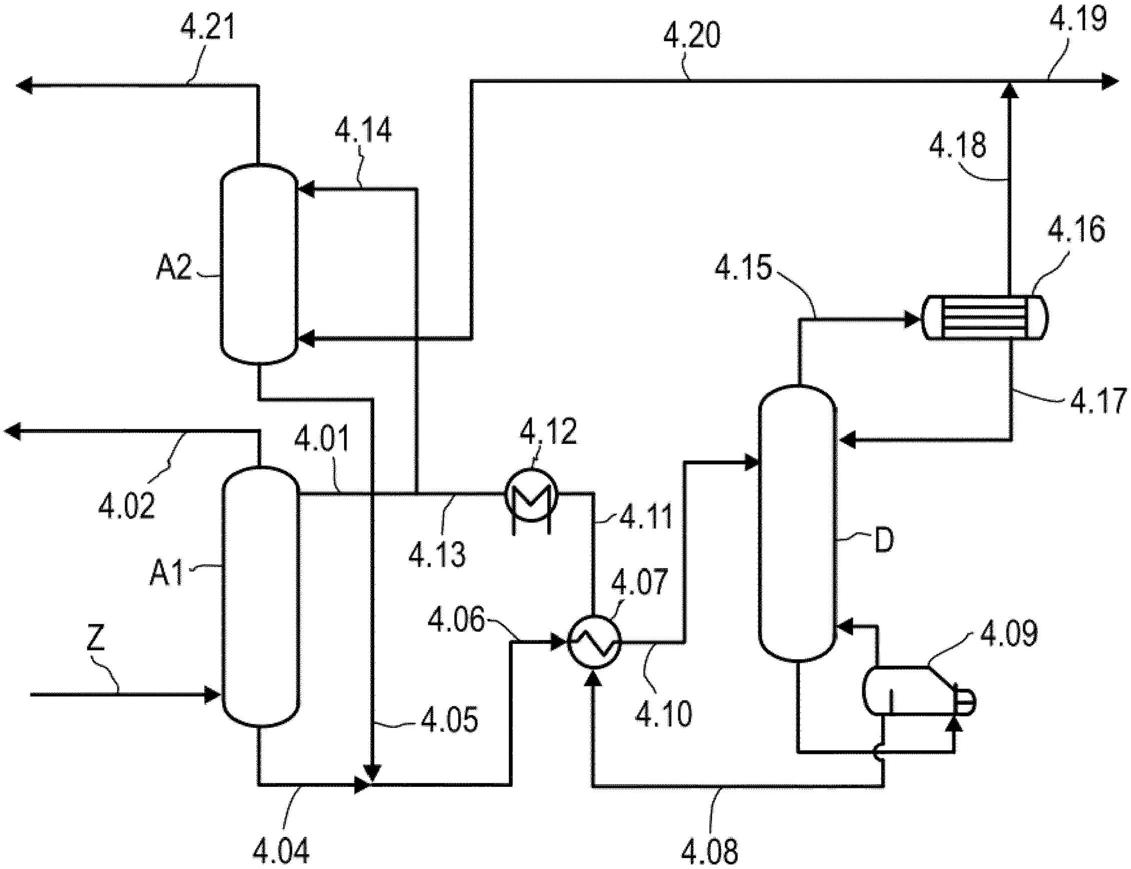
4.21
4.20
4.19
4.14
4.18
A2
4.16
4.15
4.17
4.01
4.12
4.02
4.11
D
A1
4.13
4.07
4.06
4.09
Z
4.10
4.05
4.04
4.08

Fig. 4

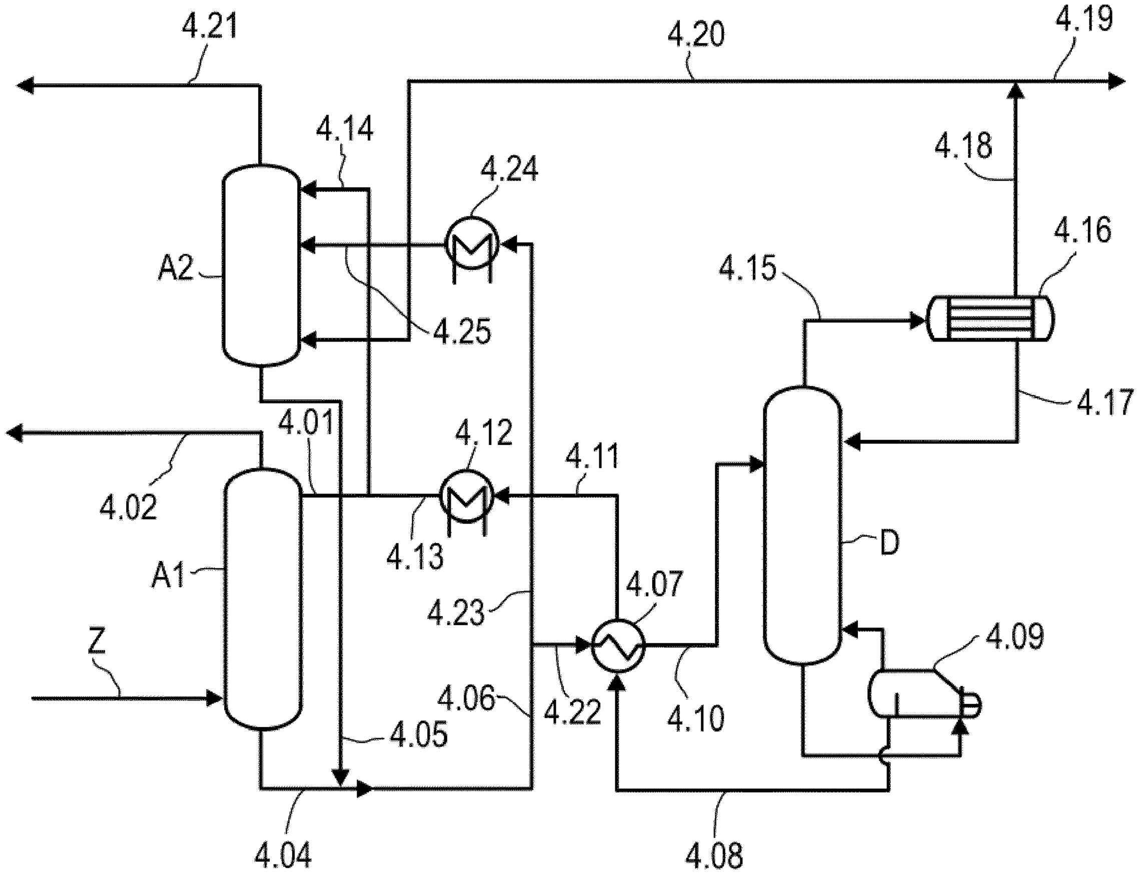
4.21
4.20
4.19
4.14
4.24
4.18
4.16
A2
4.15
4.25
4.17
4.01
4.12
4.11
4.02
4.13
D
A1
4.07
4.23
4.09
Z
4.06
4.22
4.10
4.05
4.04
4.08

Fig. 5

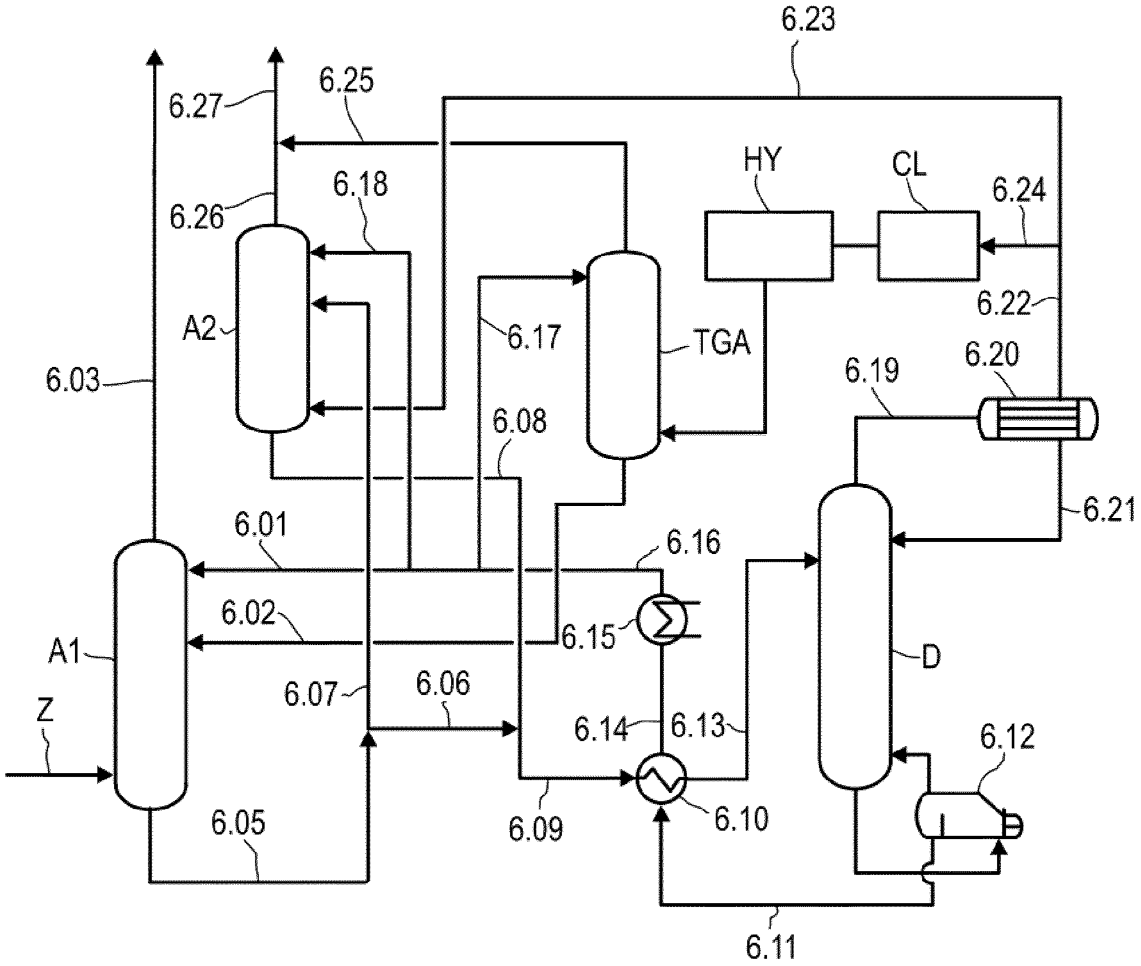
6.23
6.25
6.27
6.26
6.18
HY
CL
6.24
6.22
A2
6.17
TGA
6.03
6.19
6.20
6.08
6.01
6.16
6.21
6.02
6.15
A1
D
6.07
6.06
Z
6.14
6.13
6.12
6.05
6.10
6.09
6.11

Fig. 6

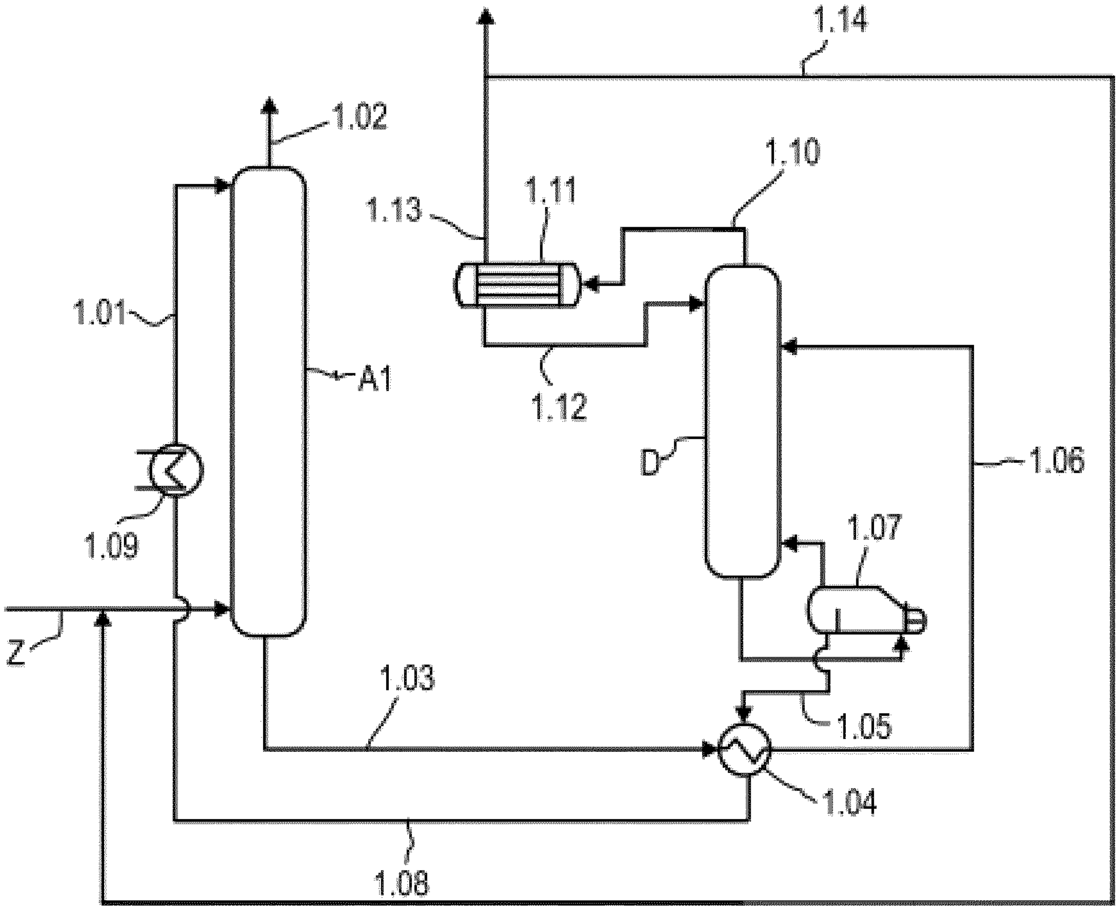


Fig. 7

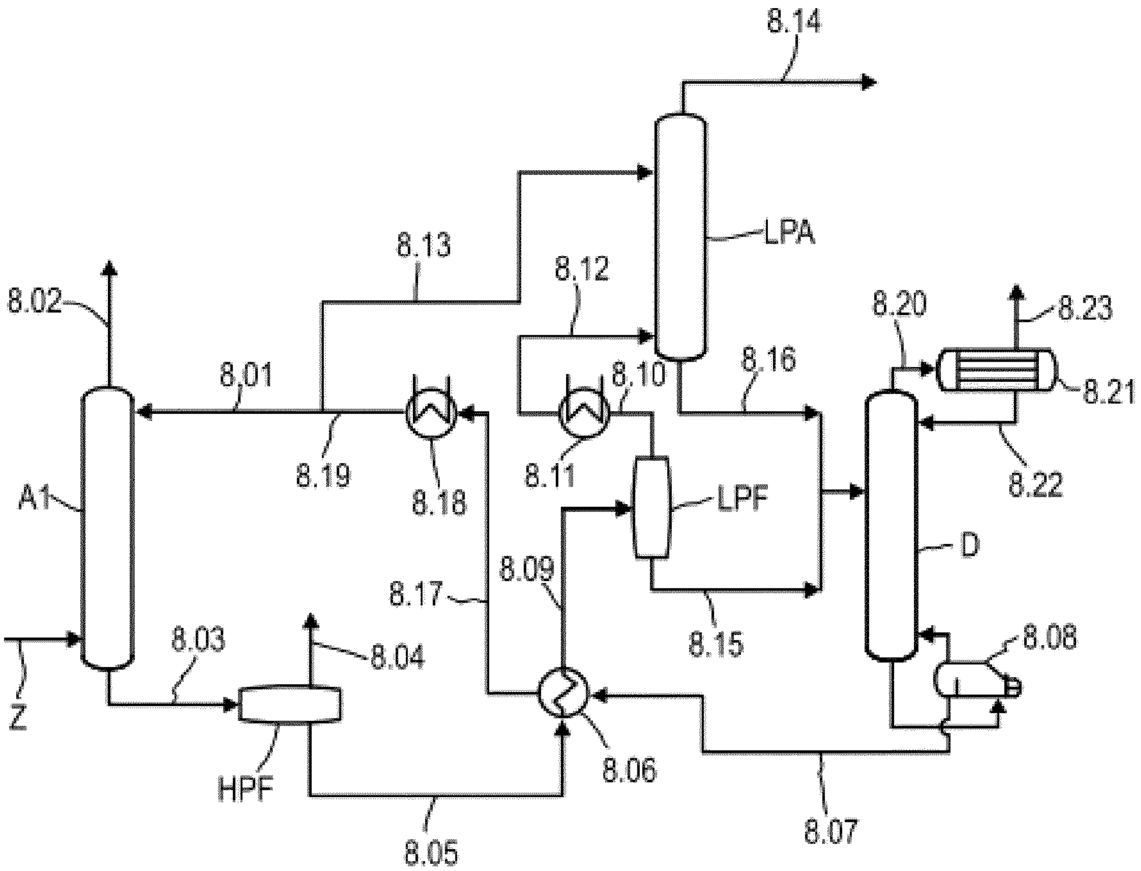
8.14
8.13
8.12
LPA
8.02
8.20
8.23
8.01
8.10
8.16
8.21
8.19
8.18
8.11
LPF
8.22
A1
D
8.09
8.17
8.15
8.08
8.03
8.04
Z
8.06
HPF
8.05
8.07

Fig. 8

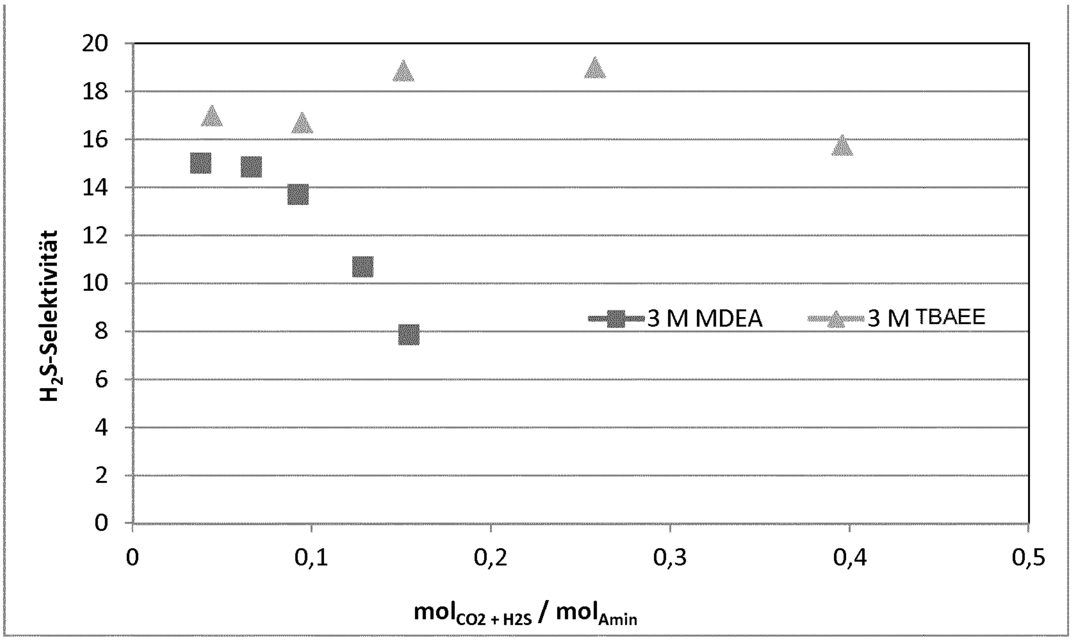
20
18
16
14
12
10
8
6
4
2
0
H2S-Selektivität
3 M MDEA
3 M TBAEE
0
0,1
0,2
0,3
0,4
0,5
molCO2 + H2S / molAmin

Fig. 9

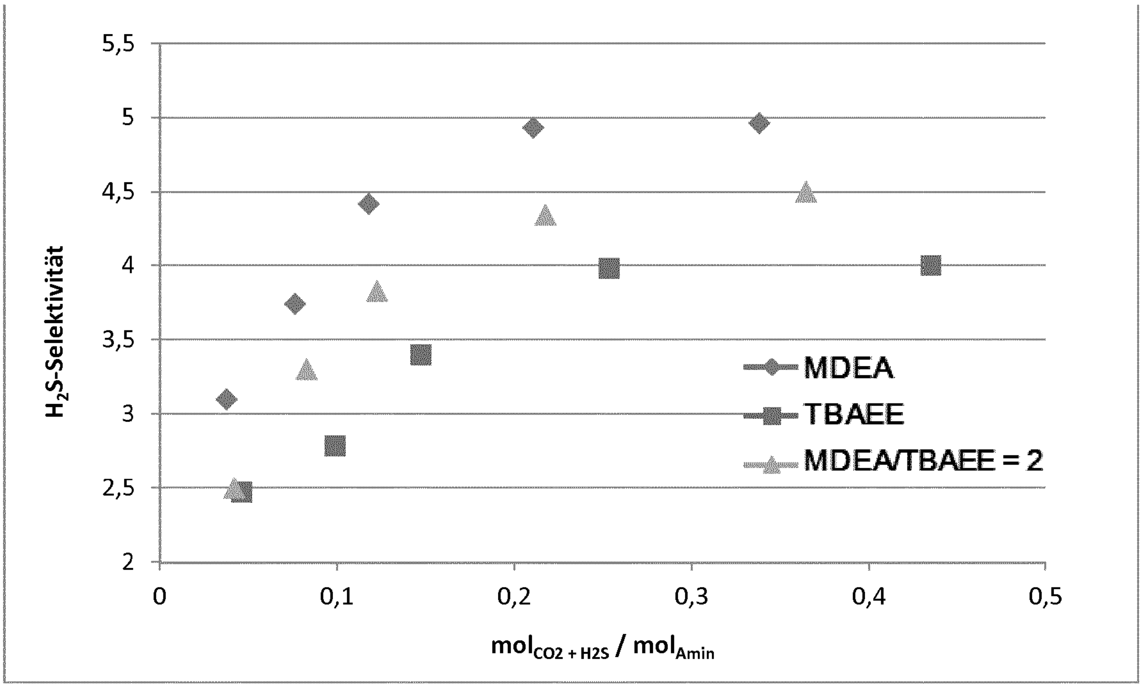
5,5
5
4,5
4
3,5
3
2,5
2
0
0,1
0,2
0,3
0,4
0,5
$H_2S$-Selektivität
$mol_{CO2 + H2S}$ / $mol_{Amin}$
MDEA
TBAEE
MDEA/TBAEE = 2

Fig. 10

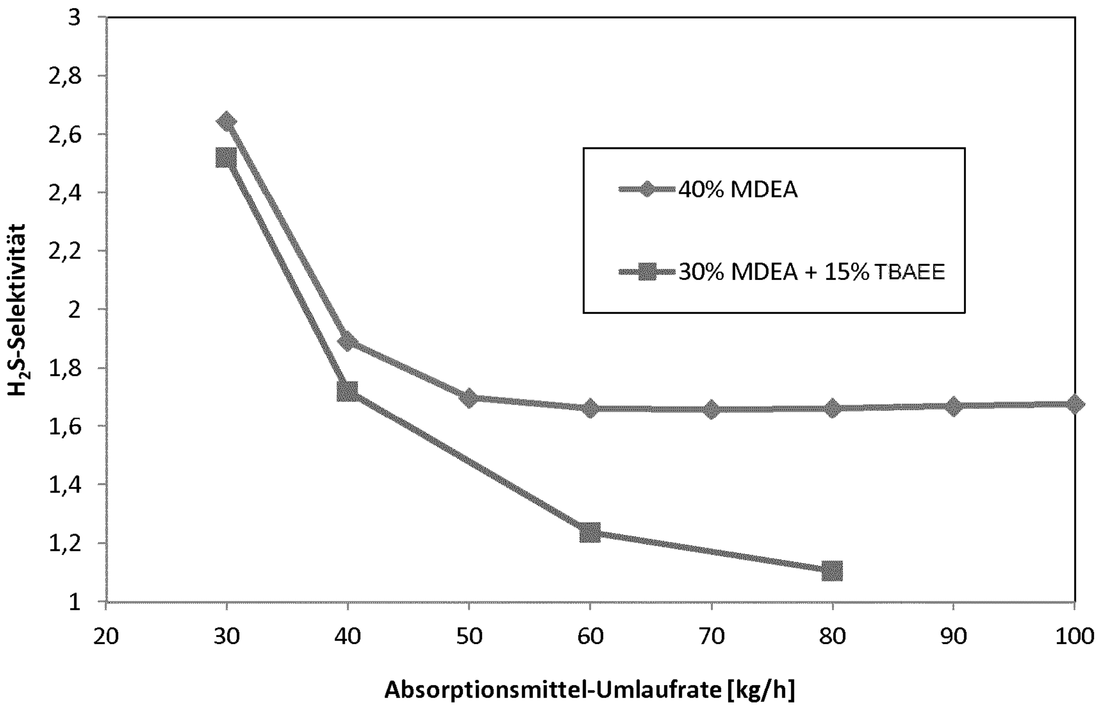
3
2,8
2,6
2,4
2,2
2
1,8
1,6
1,4
1,2
1
20
30
40
50
60
70
80
90
100
H2S-Selektivität
Absorptionsmittel-Umlaufrate [kg/h]
40% MDEA
30% MDEA + 15% TBAEE

Fig. 11

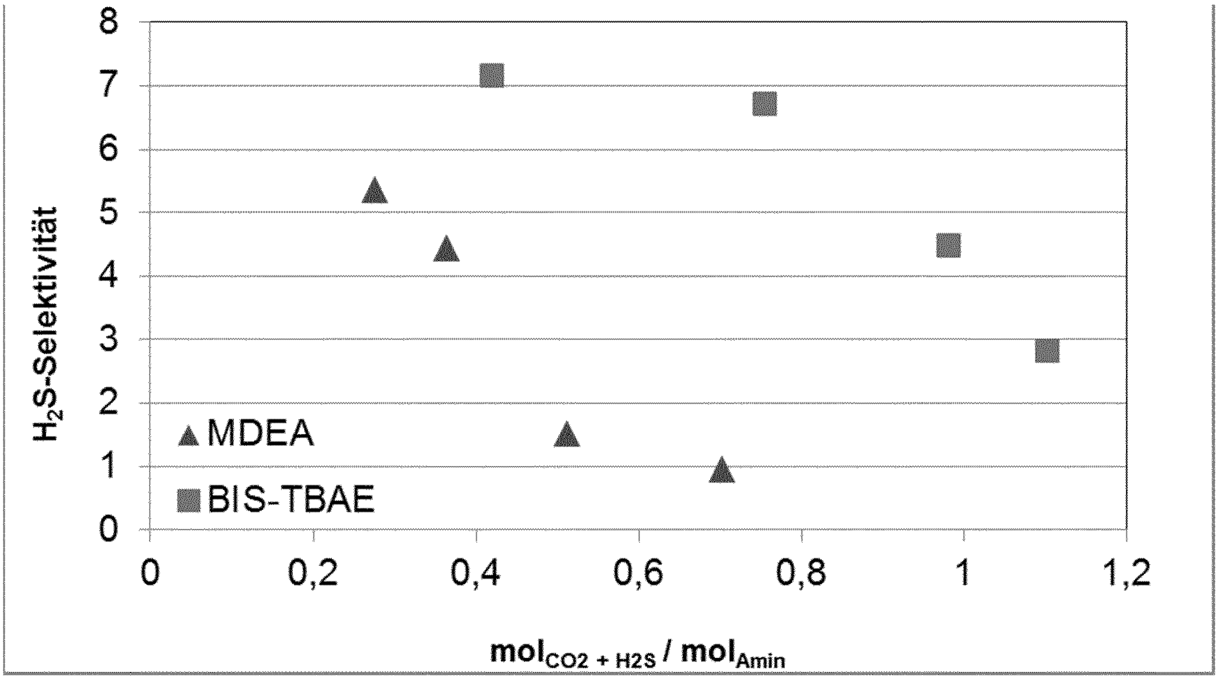
8
7
6
5
4
3
2
1
0
H2S-Selektivität
MDEA
BIS-TBAE
0
0,2
0,4
0,6
0,8
1
1,2
molCO2 + H2S / molAmin

Fig. 12

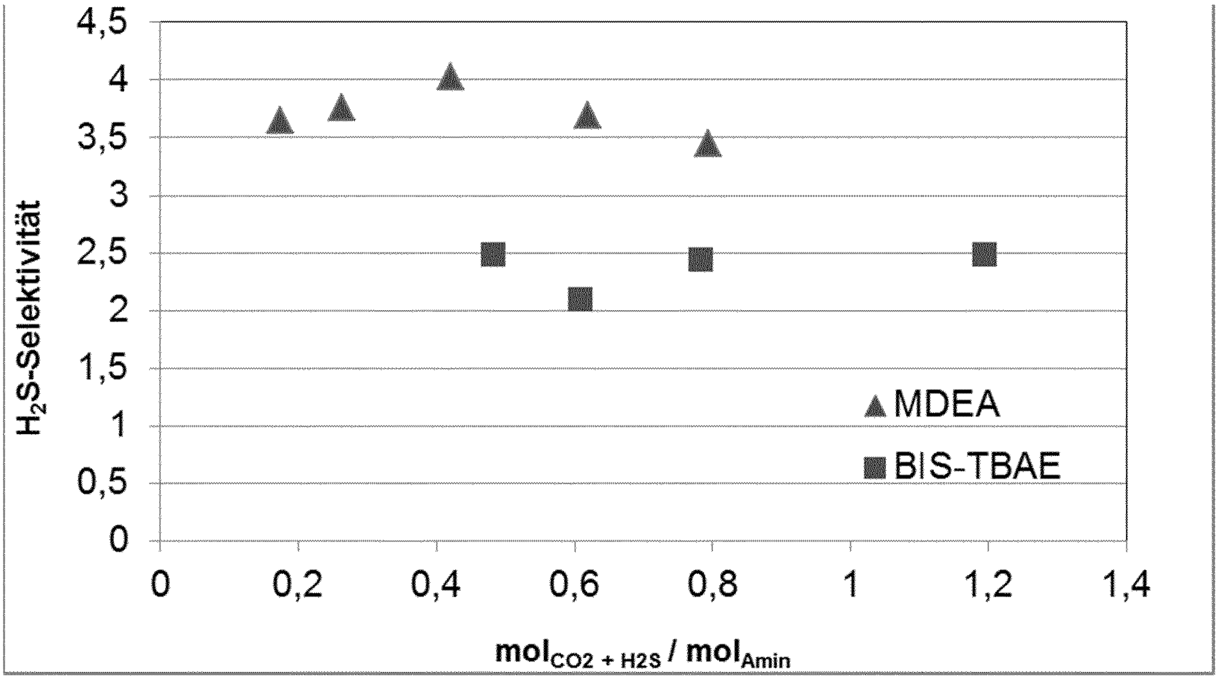
4,5
4
3,5
3
2,5
2
1,5
1
0,5
0
0
0,2
0,4
0,6
0,8
1
1,2
1,4
$H_2S$-Selektivität
MDEA
BIS-TBAE
$mol_{CO2 + H2S}$ / $mol_{Amin}$

Fig. 13

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 4471138 A
- EP 0084943 A
- US 20130243676 A
- EP 0159495 A
- EP 0190434 A
- EP 0359991 A
- WO 00100271 A
- US 4537753 A
- US 4553984 A
- EP 0202600 A
- EP 0121109 A
- EP 0084943 A2

### In der Beschreibung aufgeführte Nicht-Patentliteratur

- **LU et al.** *Separation and Purification Technology,* 2006, vol. 52, 209-217